(19) Europäisches Patentamt
European Patent Office
Office européen des brevets

(11) **EP 4 136 439 B1**

(12) **EUROPEAN PATENT SPECIFICATION**

(45) Date of publication and mention
of the grant of the patent:
**03.06.2026 Bulletin 2026/23**

(21) Application number: **21729194.7**

(22) Date of filing: **16.04.2021**

(51) International Patent Classification (IPC):
**G01N 27/74** *(2006.01)* **G01N 33/543** *(2006.01)*

(52) Cooperative Patent Classification (CPC):
**G01N 27/745; G01N 33/54326; G01N 33/56983;**
G01N 2333/165

(86) International application number:
**PCT/US2021/070401**

(87) International publication number:
**WO 2021/212144 (21.10.2021 Gazette 2021/42)**

(54) **MAGNETIC PARTICLE SPECTROSCOPY DEVICE**

VORRICHTUNG ZUR MAGNETPARTIKELSPEKTROSKOPIE

DISPOSITIF DE SPECTROSCOPIE DE PARTICULES MAGNÉTIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.04.2020 US 202063011702 P**

(43) Date of publication of application:
**22.02.2023 Bulletin 2023/08**

(73) Proprietor: **Regents of the University of
Minnesota
Minneapolis, MN 55455 (US)**

(72) Inventors:
• **WU, Kai
Saint Paul, Minnesota 55113 (US)**
• **CHUGH, Vinit Kumar
Minneapolis, Minnesota 55414 (US)**
• **WANG, Jian-Ping
Shoreview, Minnesota 55126 (US)**

(74) Representative: **Murgitroyd & Company
165-169 Scotland Street
Glasgow G5 8PL (GB)**

(56) References cited:
**EP-A1- 3 470 832        EP-A1- 3 581 916
WO-A1-2018/144599**

## Description

This application claims the benefit of U.S. Provisional Patent Application Serial No. 63/011,702, entitled "MAGNETIC PARTICLE SPECTROSCOPY METHOD AND DEVICE," and filed on April 17, 2020.

## TECHNICAL FIELD

This disclosure relates to magnetic particle spectroscopy.

## BACKGROUND

Bioassays are procedures for detecting or measuring the concentration or potency of a substance by its effect on living cells or tissues. Immunoassays are procedures for detecting or measuring specific proteins or other substances through their properties as antigens and/or antibodies. In some instances, immunoassays, among other tests, may be performed using magnetic particle spectroscopy (MPS). EP 3 470 832 A1 relates to an electromagnetic sensing device for detecting magnetic nanoparticles. EP 3 581 916 A1 relates to a device and method for magnetic particle determination. WO 2018/144599 A1 relates to magnetic nanoparticle characterization.

## SUMMARY

In general, this disclosure describes example magnetic particle spectroscopy (MPS) devices and techniques for detecting chemicals and biological substances via MPS.

In some examples, a handheld MPS device is described. The handheld MPS device may be configured to generate an alternating magnetic field including a plurality of frequencies via conductive excitation coils. The handheld MPS device may be configured to position a sample including surface functionalized magnetic nanoparticles (MNPs) within the conductive excitation coils and within the alternating magnetic field. The handheld MPS device may be further configured to determine a magnetic response of the surface functionalized MNPs to the alternative magnetic field via a conductive sensing coil, e.g., a pick-up coil, which may generate a signal based on the magnetic response. In some examples, a biological sample may be added to the sample including the surface functionalized MNPs, and analytes within the biological sample may bind with the surface functionalized MNPs thereby changing the hydrodynamic size of the surface functionalized MNPs and, consequently, the magnetic response of the surface functionalized MNPs to the alternating magnetic field.

In some examples, a microfluidic MPS device is described. The microfluidic MPS device may be configured to be in fluid communication with a microfluidic filtering device configured to filter a fluid, e.g., blood, mix a fluid including analytes from the filtered fluid with a fluid including surface functionalized MNPs via microfluidic mixing channels, and provide the mixed fluid to a plurality of microfluidic channels. The microfluid MPS device may include a microfluidic sample strip including a plurality of sensing regions and in fluidic communication with one of the plurality of microfluidic channels of the microfluidic filtering device. Each sensing region may include conductive excitation coils and conductive sensing coils, and a surface area functionalized with biologic probes configured to bind with a specific analyte type. The sensing coils of each sensing region may detect the magnetic response of surface functionalized MNPs captured via binding to analytes that are bound to probes on the sensing region surface to an alternating magnetic field including a plurality of frequencies generated via the conductive excitation coils, and the sensing coils may output a signal proportional to the detected magnetic response. In some examples, the microfluidic MPS device may include a fluid container rather than a microfluidic sample strip. The fluid container may include one or more inner surfaces functionalized via coating of biologic probes configured to bind to a specific analyte type. The fluid container may be in fluidic communication with one of the plurality of microfluidic channels of the microfluidic device. The fluid container may include, and be disposed within, conductive excitation coils for generating an alternating magnetic field including a plurality of frequencies and conductive sensing coils for detecting the magnetic response of magnetic nanoparticles placed within the fluid container and bound to the specific analyte type bound to the surface via binding with the biologic probes.

Accordingly, the techniques disclosed may provide one or more technical advantages. For example, the techniques may provide a one step, wash-free immunoassay with reduced technical requirements. In examples, the techniques may improve sensitivity, improve portability, reduce cost and maintenance, reduce sample preparation time and complexity, reduce reliance on skilled technicians, may be performed in vitro, and may provide a point-of-care (POC) detection kit with improved ease of use.

In some examples, the disclosure describes a bioassay system comprising: at least one conductive excitation coil, the at least one conductive excitation coil configured to generate an alternating magnetic field including a first frequency and a second frequency; a sample mount configured to position a sample within the at least one conductive excitation coil; and at least one sensing conductive coil configured to determine a magnetic response of a sample positioned within the sample mount to the alternating magnetic field.

In some examples, the disclosure describes a bioassay system comprising: a first sample region comprising: at least one first conductive excitation coil, the at least one first conductive excitation coil configured to generate an alternating magnetic field including one of at least one sinusoidal wave, a square wave, a triangular wave, a sawtooth wave, and combination of sinusoids thereof; a first surface within the first sample region, the first surface coated with at least one probe configured to capture a first analyte type; and at least one first conductive sensing coil configured to determine a magnetic response of a surface functionalized MNP configured to be captured by the first analyte type; a second sample region comprising: at least one second conductive excitation coil, the at least one second conductive excitation coil configured to generate an alternating magnetic field including one of at least one sinusoidal wave, a square wave, a triangular wave, a sawtooth wave, and combination of sinusoids thereof; a second surface within the second sample region, the second surface coated with at least one probe configured to capture a second analyte type; at least one second conductive sensing coil configured to determine a magnetic response of a surface functionalized MNP configured to be captured by the second analyte type.

In some examples, the disclosure describes a bioassay system comprising: at least one conductive excitation coil, the at least one conductive excitation coil configured to generate an alternating magnetic field including a plurality of frequencies; a sample mount configured to position a sample within the at least one conductive excitation coil; and at least one sensing conductive coil configured to determine a magnetic response of a sample positioned within the sample mount to the alternating magnetic field.

Thus, the disclosed embodiments provide MPS techniques for one step, wash-free immunoassays with improved portability and sensitivity and reduced cost and complexity.

## BRIEF DESCRIPTION OF THE DRAWINGS

FIG. 1 is an illustration of an example diagnosis system including an MPS handheld device, in accordance with one or more techniques of this disclosure.

FIG. 2 is a block diagram of example circuit of an example MPS handheld device, in accordance with one or more techniques of this disclosure.

FIG. 3 is a block diagram of another example circuitry of an example MPS handheld device, in accordance with one or more techniques of this disclosure.

FIG. 4 is a flowchart of an example method of measuring a sample using MPS handheld device, in accordance with one or more techniques of this disclosure.

FIG. 5 is an illustration of an example series of user interface screens of a mobile application, in accordance with one or more techniques of this disclosure.

FIG. 6 is a plan view of microfluidic device, in accordance with one or more techniques of this disclosure.

FIG. 7 is cross-sectional diagram of a portion of microfluidic device, in accordance with one or more techniques of this disclosure.

FIG. 8 is cross-sectional diagram of a portion of microfluidic device, in accordance with one or more techniques of this disclosure.

FIG. 9 is an illustration of example of fluid filtration of a microfluidic device, in accordance with one or more techniques of this disclosure.

FIG. 10 is an illustration of example mixing microfluidic channels of microfluidic device, in accordance with one or more techniques of this disclosure.

FIG. 11A is an illustration of another example of mixing microfluidic channels that may be used in microfluidic device, in accordance with one or more techniques of this disclosure.

FIG. 11B is an of a portion of microfluidic device, in accordance with one or more techniques of this disclosure.

FIG. 12A is a schematic cross-sectional illustration of a portion of an example MPS handheld device, in accordance with one or more techniques of this disclosure.

FIG. 12B is an illustration of an example plot of the amplitude of a composite excitation magnetic field as a function of frequency, in accordance with one or more techniques of this disclosure.

FIG. 12C is an illustration of an example plot of the amplitude of magnetic responses from MNPs detected by pick-up coils as a function of frequency, in accordance with one or more techniques of this disclosure.

FIG. 13A is an illustration an example plot of the amplitude of an alternating magnetic field generated by drive coils as a function of time, in accordance with one or more techniques of this disclosure.

FIG. 13B is an illustration an example plot of the amplitude of composite magnetic field as a function of frequency, in accordance with one or more techniques of this disclosure.

FIG. 13C is an illustration of an example plot of the magnetic response of MNPs as a function of magnetic field, in accordance with one or more techniques of this disclosure.

FIG. 13D is an illustration an example plot of the amplitude of a magnetic response of one or more MNPs within the generated magnetic field of FIG. 13A as a function of time, in accordance with one or more techniques of this disclosure.

FIG. 13E is an illustration of an example plot of the amplitude of the magnetic response of FIG. 13D as a function of frequency, in accordance with one or more techniques of this disclosure.

FIG. 14A is an illustration of an example plot of the harmonic amplitude response as a function of hydrodynamic size of MNPs, in accordance with one or more techniques of this disclosure.

FIG. 14B is an illustration of an example plot of the harmonic amplitudes of different types of MNPs, in accordance with one or more techniques of this disclosure.

FIG. 14C is an illustration of an example plot of the harmonic amplitudes of different amounts of a type of MNP, in accordance with one or more techniques of this disclosure.

FIG. 14D is an illustration of an example plot of the harmonic amplitudes of an MPS measurement of streptavidin, in accordance with one or more techniques of this disclosure.

FIG. 15 is an illustration of an example surface functionalized MNP, in accordance with one or more techniques of this disclosure.

FIG. 16 is an illustration of example surface functionalized MNPs, in accordance with one or more techniques of this disclosure.

FIG. 17 is an illustration of an example surface functionalized MNP, in accordance with one or more techniques of this disclosure.

FIG. 18 is an illustration of an example surface functionalized MNP, in accordance with one or more techniques of this disclosure.

FIG. 19 is an illustration of an example surface functionalized MNP, in accordance with one or more techniques of this disclosure.

FIG. 20 is an illustration of example surface functionalized MNPs, in accordance with one or more techniques of this disclosure.

FIG. 21 is an illustration of an example surface functionalized MNP, in accordance with one or more techniques of this disclosure.

FIG. 22 is an illustration of example surface functionalized MNPs, in accordance with one or more techniques of this disclosure.

FIG. 23 is an illustration of an example surface functionalized MNPs, in accordance with one or more techniques of this disclosure.

FIG. 24 is an illustration of example surface functionalized microbead and MNP, in accordance with one or more techniques of this disclosure.

FIG. 25A is an illustration of example surface functionalized microbead and MNP, in accordance with one or more techniques of this disclosure.

FIG. 25B is an illustration of an example surface functionalized MNP, in accordance with one or more techniques of this disclosure.

FIG. 25C is an illustration of an example surface functionalized MNP, in accordance with one or more techniques of this disclosure.

FIG. 26 is an illustration of an exploded-view of an example MPS device, in accordance with one or more techniques of this disclosure.

FIG. 27 is an illustration of an example MPS device, in accordance with one or more techniques of this disclosure.

FIG. 28 is an illustration of an example microfluidic system that may be used with an MPS device, in accordance with one or more techniques of this disclosure.

FIG. 29 is a flowchart of an example method of measuring a sample using MPS devices, in accordance with one or more techniques of this disclosure.

FIG. 30 is an illustration of example MPS devices, in accordance with one or more techniques of this disclosure.

Like symbols in the drawings indicate like elements.

## DETAILED DESCRIPTION

In recent years, magnetic particle spectroscopy (MPS) has emerged as a new technology for immunoassay applications. In MPS, alternating magnetic fields may be applied to magnetic nanoparticles (MNPs). The magnetic responses of these nanoparticles may be collected and recorded by a pair of specially designed pick-up coils. These magnetic responses may contain higher harmonics that are specific to the physical changes of the nanoparticles, such as binding events of target analytes to nanoparticles. MPS may be a volumetric-based bioassay method that analyzes the response signal from the whole nanoparticle suspension, and may allow for one step, wash-free immunoassay with reduced technical requirements. In examples, a handheld MPS system that may have high, or increased, sensitivity, reduced cost, may be performed in vitro, and may be an easy-to-use point-of-care (POC) detection kit is disclosed.

Magnetic particle imaging (MPI) has emerged as a new imaging modality that directly detects MNP tracers using alternating magnetic fields. MPS, a derivative technology of MPI, has emerged as a novel immunoassay tool that may be a wash-free, easy-to-use, and portable home healthcare modality. MPS may be interpreted as 0D MPI, e.g., a spatially independent MPI, where a bi-directional sinusoidal magnetic field may be applied to a suspension of MNPs. The magnetization of MNPs relax in response to the external magnetic field through Brownian and Néel relaxations. These MNPs may be specially designed and coated with antibodies that specifically recognize and bind to target analytes from biofluid samples. Due to the nonlinear magnetic responses of these MNPs, higher harmonics may be picked up by a pair of specially designed pick-up coils. These harmonics may be extracted by means of appropriate filtering and may be indicators of the physical environments of MNPs, for example, the viscosity and temperature of the liquid medium as well as the binding events of target analytes onto MNPs. Since biological tissues and fluids are nonmagnetic, there may be negligible magnetic background noise from biological samples. MNPs may be the sole sources of magnetic responses from testing samples and this volumetric-based immunoassay tool may allow for reduced sample preparation.

Conventional disease diagnosis systems are often bulky in size, expensive, and rely on the expertise of skilled technicians as well as daily equipment maintenance. Such systems may not be used widely as advanced immunoassay technologies. In addition, disease diagnosis may often be unaffordable, for example, for people living in poor regions and developing countries. In some examples, the current disclosure describes MPS handheld devices for early diagnosis of diseases that may be portable and cost-effective. In some examples, the MPS handheld devices may mitigate the health burden in poor and developing regions and may enable early diagnosis of diseases, which may effectively prevent the spread of infectious diseases and save lives. In some examples, the MPS handheld devices may be used indoors or outdoors, for example, at research centers, medical clinics, homes, and in the field. In some examples, the MPS handheld device may provide users with an inexpensive, fast, convenient, and easy-to-use monitoring system. For example, the MPS handheld devices may allow users to perform daily monitoring of chronic diseases. In some implementations, the MPS handheld device may provide clinics, hospitals, and pharmaceutical industries with reduced laboratory and maintenance expenses. The MPS handheld devices may additionally provide versatile bio-sensing ability with a simple process, portability, and the ability to communicate wirelessly with mobile devices. In some examples, after receiving user consent, the MPS handheld devices may enable users to share real-time and long-term monitoring information with a medical provider and empower remote disease diagnostics, and may enable sharing of real-time information with local, regional, state, and/or federal entities, such as the Centers for Disease Control, for real-time monitoring of the spread and/or overspread of infectious disease. The MPS handheld devices may be a platform used for different disease detection and multiplexed immunoassays using just one sample (e.g. a drop of body fluid) in a single test.

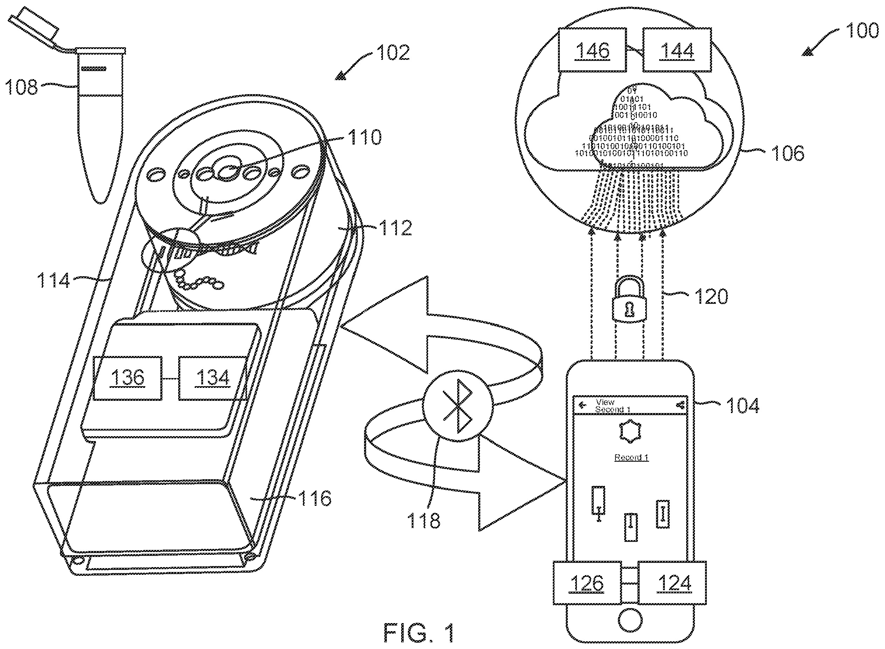

FIG. 1 is an illustration of an example diagnosis system 100 including an MPS handheld device 102, in accordance with one or more techniques of this disclosure. In the example shown, diagnosis system 100 includes MPS handheld device 102, a computing device 104, a distributed computing system 106, e.g., cloud computing system 106, and a sample vial 108.

In the example shown, MPS handheld device 102 includes a sample loading port 110, coils 112, a housing 114, and circuitry 116. Sample loading port 110 may be configured to accept samples, for example, sample vial 108, and to position the sample to be tested in the correct position with respect to coils 112 for an MPS measurement or measurements. Coils 112 may include drive coils, e.g., primary coils, secondary coils, etc., and pick-up coils, or any type of coil suitable for forming, controlling, and detecting or sensing magnetic fields. Coils 112 may be made of any suitable conductive and/or magnetic material, for example, copper, silver, aluminum, or the like. Housing 114 may be configured to enclose, support, and position the components of MPS handheld device 102 correctly with respect to each other, e.g., sample loading port 110 and coils 112, to provide a structure for connecting circuitry 116 to coils 112 and any other components of MPS handheld device 102, and to provide structure for a user physically manipulate MPS handheld device 102. In some examples, housing 114 may be 3D printed with any suitable material, such as a polymer. In some examples, the polymer may include polylactic acid (PLA). In some examples, MPS handheld device 102 may be manipulatable by hand by a user, e.g., MPS handheld device 102 may be a handheld device.

In some examples, sample vial 108 may include or contain a liquid including one or more MNPs. In some examples, material to be tested, e.g., a fluid such as a sample of a patient's blood or blood components, may be added to sample vial 108. The MNPs included in sample vial 108 may be surface functionalized via coating with ligands (e.g., carboxylic acid and amine, and the like), proteins (e.g., antibodies, streptavidin, protein A, and the like), antigens, nucleic acids (e.g., deoxyribonucleic acid (DNA), ribonucleic acid (RNA), or the like) or any combination thereof, or may be carried in a liquid. Sample vial 108 may be configured to have a long shelf life, for example, a shelf life greater than one hour, greater than one day, greater than one week, greater than one month, greater than one year, greater than ten years, or any other long shelf life. In some examples, the MNPs included in sample vial 108 may be configured to be stored at room temperature, or at lower temperatures, for example, near 4° Celsius (C). The MNPs included in sample vial 108 may be configured to be surface functionalized with different antibodies, antigens, DNA, RNA, and the like, designed to detect one or more specific biomarkers, e.g., one or more specific disease. In some examples, sample vial 108 may be a flat bottom, USP type I glass vial, may have dimensions of 31 millimeters (mm) by 5 mm and a volume capacity of 0.25 milliliters (mL), and may be one-time use only, e.g., disposable. In other examples, sample vial 108 may be a plastic vial, or made of any other suitable material.

In some examples, MPS handheld device 102 may be communicatively coupled, for example by a wired or a wireless connection 118 and/or 120, to computing device 104 and/or distributed system 106. In some examples, connection 118 and/or 120 may be a secured connection, e.g., encrypted, requiring two-factor authentication, and the like. Measurements and/or information corresponding to measurements may be transferred to computing device 106 and/or distributed system 106, for example, for processing of measurements and/or information corresponding to measurements. In some examples, circuitry 116 of MPS handheld device 102 may include processing circuitry 136 and memory 134, and may process measurements and/or information corresponding to measurements without transferring the measurements and/or information corresponding to measurements to computing device 104 or distributed computing system 106. In some examples, computing device 104 may be communicatively coupled to distributed computing system 106, for example by a wired or a wireless connection 120, and measurements and/or information corresponding to measurements from MPS handheld device 102 received by computing device 104 may be transferred to distributed computing system 106, for example, for processing of measurements and/or information corresponding to measurements.

In the illustrated example, computing device 106 may include processing circuitry 126 coupled to memory 124 and to a display, one or more outputs, and one or more user inputs of a user interface. Processing circuitry 126, as well as processing circuitry 136, and other processing modules or circuitry described herein, e.g., processing circuitry 146 of distributed computing system 106, may be any suitable software, firmware, hardware, or combination thereof. Processing circuitry 126, 136, 146 may include any one or more microprocessors, controllers, digital signal processors (DSPs), application specific integrated circuits (ASICs), field-programmable gate arrays (FPGAs), or discrete logic circuitry. The functions attributed to processors described herein, including processing circuitry 126, may be provided by processing circuitry of a hardware device, e.g., as supported by software and/or firmware.

In some examples, processing circuitry 126, as well as processing circuitry 136, 146, may be configured to determine diagnosis information associated with MPS measurements and/or MPS measurement information. For example, the processing circuitry 126 may determine amplitudes and/or phases of magnetic fields detected via coils 112 and may perform any suitable signal processing to determine a diagnosis based on the amplitudes and/or phases of the magnetic fields. Processing circuitry 126 may also receive input signals from additional sources (not shown). For example, processing circuitry 126 may receive an input signal containing position information, such as Global Navigation Satellite System (GNSS) coordinates of MPS handheld device 102 and/or computing device 104. Additional input signals may be used by processing circuitry 126 in any of the calculations or operations it performs. In some examples, processing circuitry 126 may be adapted to execute software, which may include an operating system and one or more applications, as part of performing the functions described herein. In some examples, processing circuitry 126 may include one or more processing circuitry modules for performing each or any combination of the functions described herein.

In some examples, processing circuitry 126 may be coupled to memory 124, processing circuitry 136 may be coupled to memory 134, and processing circuitry 146 may be coupled to memory 144. Memory 124, as well as memory 134 and 144, may include any volatile or non-volatile media, such as a random-access memory (RAM), read only memory (ROM), non-volatile RAM (NVRAM), electrically erasable programmable ROM (EEPROM), flash memory, and the like. Memory 124, 134, and 144 may be a storage device or other non-transitory medium. Memory 124, 134, and 144 may be used by processing circuitry 126, 136, and 146, respectively, for example, to store information corresponding MPS handheld device 102 measurements. In some examples, processing circuitry 126, 136, and 146 may store measurements or previously received data in memory 124, 134, and 144, respectively, and/or calculated values for later retrieval. In some examples, MPS handheld device 102 may be powered by a wall-plug, e.g., via alternating current (AC) power and may include power circuitry such as an AC adapter. In some examples, MPS handheld device 102 may be alternatively and/or additionally powered by batteries, solar cells, or any other suitable power source.

Processing circuitry 126 may be coupled to a user interface including a display, user inputs, and outputs. In some examples, the display may include one or more display devices (e.g., monitor, personal digital assistant (PDA), mobile phone, tablet computer, any other suitable display device, or any combination thereof). For example, the display may be configured to display measurements, measurement information, and/or diagnosis information. In some examples, the user input is configured to receive input from a user, e.g., information corresponding to MPS handheld device 102, a patient, and/or a sample, e.g., sample vial 108. For example, a user may input information such as MPS handheld device 102 parameters or sample vial 108 information by manually entering the product number from MPS handheld device 102 or sample vial 108, or by scanning a quick response (QR) code/bar code from MPS handheld device 102 or sample vial 108. In some examples, MPS handheld device 102 and/or sample vial 108 may be labeled with a serial number as well as a QR code or bar code for a user to input information before testing.

The user input may include components for interaction with a user, such as a keypad and a display, which may be the same as the display. In some examples, the display may be a cathode ray tube (CRT) display, a liquid crystal display (LCD) or light emitting diode (LED) display and the keypad may take the form of an alphanumeric keypad or a reduced set of keys associated with particular functions. The user input, additionally or alternatively, may include a peripheral pointing device, e.g., a mouse, via which a user may interact with the user interface. In some examples, the displays may include a touch screen display, and a user may interact with the user input via the touch screens of the displays. In some examples, the user may also interact with the user input remotely via a networked computing device.

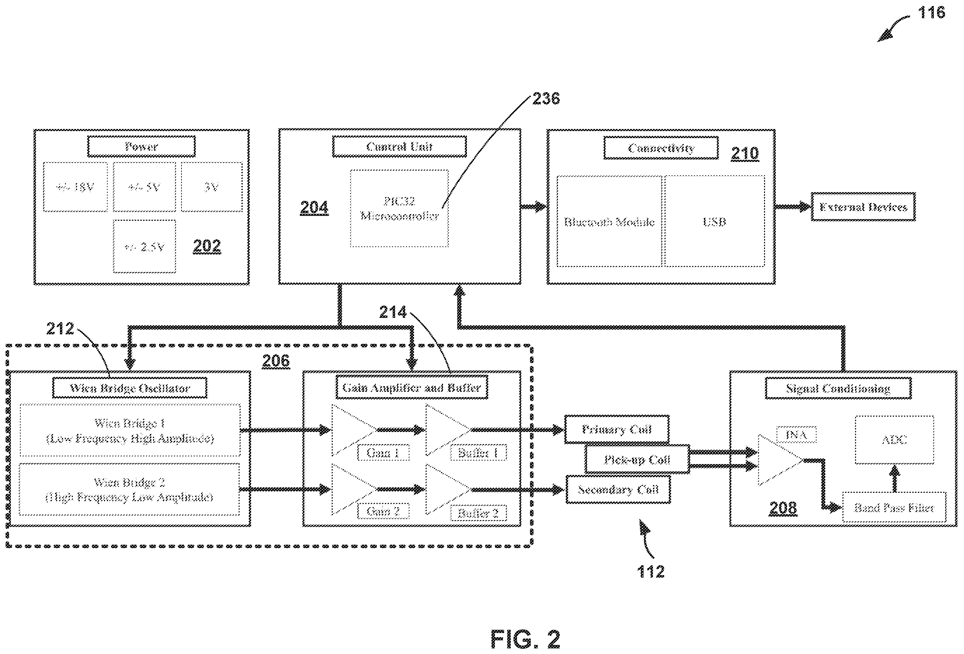

FIG. 2 is a block diagram of example circuitry 116 of an example MPS handheld device 102, in accordance with one or more techniques of this disclosure. In the example shown, circuitry 116 includes power unit 202, control unit 204, coil driver 206, signal conditioning unit 208, connectivity unit 210, and coils 112.

In the examples shown, power unit 202 is configured to generate electrical power, for example, usable direct current (DC) voltages, from an off-board alternating current (AC) power supply to be used by multiple digital and analog system components. Power unit 202 may comprise an off-board AC to DC power supply adapter to provide DC power to MPS handheld device 102. In some examples, the DC voltage may be further dropped down using high current rated linear drop-off (LDO) regulators and switching power supply components for providing a stable supply at suitable voltages to be used by different stages of MPS handheld device 102. For example, an LDO may be utilized to generate a +/-2.5V supply voltage, or a +/-5V supply voltage, or a +3.3V supply voltage, or any other suitable supply voltage to power an onboard microcontroller. In some examples, switching regulators may be used to generate -15V and +15V supply voltages to feed the coils 112, as described further below. In other examples, power unit 202 may include a battery or other portable power source and associated voltage regulation circuitry.

In some examples, control unit 204 may include microcontroller 236. In some examples, microcontroller 236 may include built-in floating-point hardware and may be utilized as an on-board processor, e.g., as processing circuitry 136. In some examples, microcontroller 236 may communicate with an analog-to-digital converter (ADC) and a communication connection module, e.g., using a using a serial peripheral interface (SPI) protocol. Microcontroller 236 may be additionally configured to select variable frequencies for coils 112, e.g., the primary and secondary drive coils, via SPI protocol to communicate with digital potentiometers for selecting appropriate excitation frequencies.

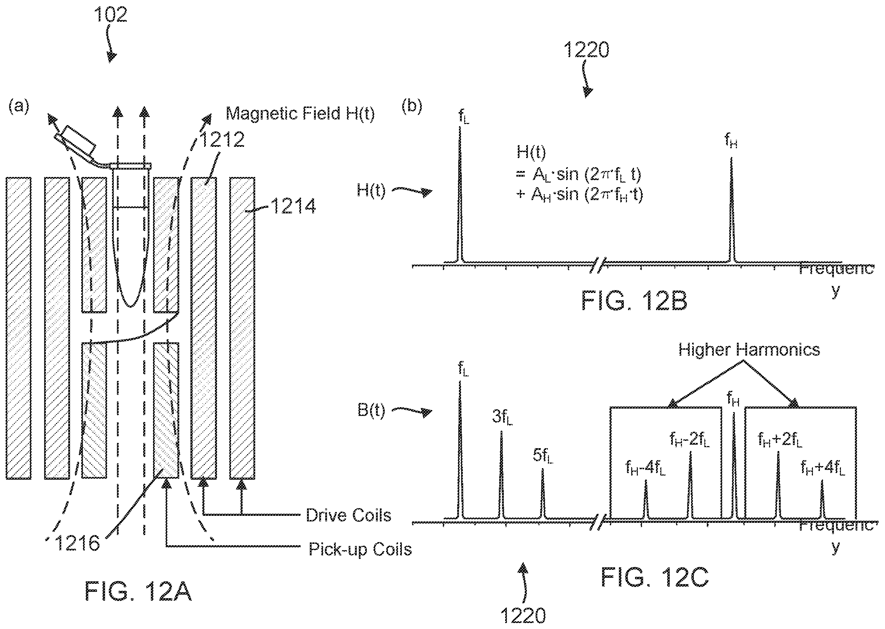

In the example shown, coil driver 206 may be configured to generate variable frequency waveforms for driving coils of coils 112. For example, coil driver 206 may generate two waveforms for primary and secondary drive coil excitation. In some examples, coil driver 206 may generate variable frequency waveforms via two sub-stage units, e.g., Wien-Bridge Oscillator 212 and gain amplifier and buffer 214. Wien-Bridge Oscillator 212 may generate base waveform signals to be further processed by later stages. Gain amplifier and buffer 214 may amplify incoming waveforms and may provide a buffer to meet high current requirements of coils 112. In some examples, waveforms may include any of a sinusoidal waveform, a rectangular or square waveform, a triangular waveform, a sawtooth waveform, or any combination thereof. In some examples, Wien-Bridge Oscillator 212 and gain amplifier and buffer 214 may include corresponding sets of circuitries for generating a low frequency high amplitude waveform for output to a primary coil and a for generating a high frequency low amplitude waveform for output to a secondary coil. For example, MPS handheld device 102 may include primary coils 112 (e.g., which may be drive coils 1212 illustrated and described below with reference to FIG. 12A) comprising 1278 turns of 13 American Wire Gage (AWG) copper wire for the low frequency high amplitude waveform, secondary coils 112 (e.g., drive coils 1214 of FIG. 12A below) comprising 449 turns of 30 AWG copper wire for the high frequency low amplitude waveform, and pick-up coils 112 (e.g., pick-up coils 1216 of FIG. 12A below) comprising 36AWG copper wire.

In some examples, coil driver 206 driving coils of coils 112 may be configured to generate phase stable magnetic fields.

In the example shown, signal conditioning unit 208 may be configured to remove (e.g., filter) noise and amplify a signal received from pick-up coils of coils 112. For example, one or more pick-up coils of coils 112 may generate a differential voltage output, and signal conditioning unit 208 may condition the differential voltage output to remove noise and amplify the differential output. In some examples, signal conditioning unit 208 may provide an initial gain and convert the differential signal from pick-up (e.g., search) coils to a single-ended signal for further processing by filtering stages. In some examples, signal conditioning unit 208 may be configured to provide Sallen-Key based second-order low-pass and highpass filtering to remove the powerline and high-frequency noises. In some examples, a cutoff frequency of a band-pass filter may be set at or near 53 kilohertz (kHz) for the low pass filtering, e.g., as the high frequency cutoff, and at or near 730 Hz for high pass filtering, e.g., as the low frequency cutoff. The filtered signal may be sampled at, for example, 200 kilo-samples per second (ksps) having 16-bit samples via an analog-to-digital (ADC) in communication with microcontroller 236 using SPI protocol. In some implementations, microcontroller 236 may transmit data sampled by the ADC via connectivity unit 210.

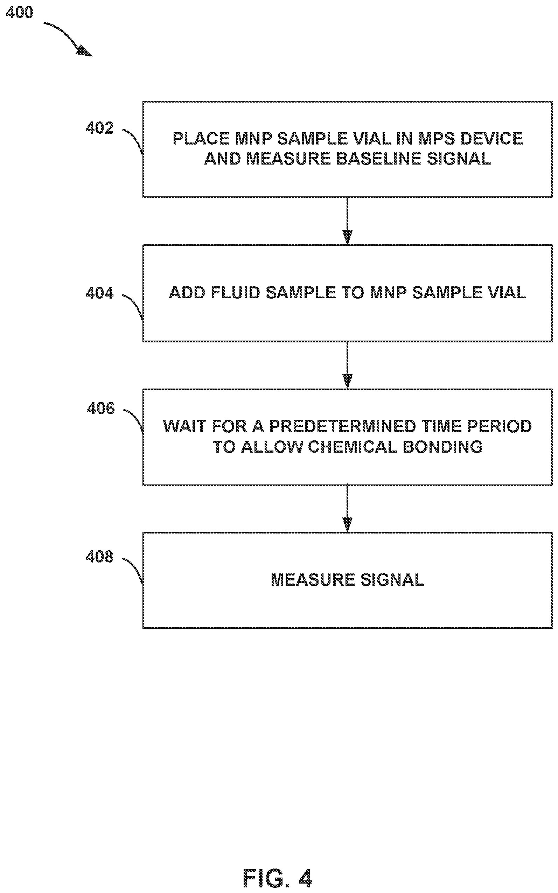

In the example shown, connectivity unit 210 may be configured to transfer data via a wired or wireless connection, e.g., connection 118. In some examples, connectivity unit 210 may communicate using any wired or wireless communication modality, for example, serial, universal serial bus (USB), WiFi, local area network (LAN), Bluetooth®, and the like. In some examples, connectivity unit 210 may be configured to execute application software. For example, application software may include a user interface configured to initiate execution of processing of information via microcontroller 236 and display of the processed information in real-time. Application software may further be configured to guide users on how to use MPS handheld device 102, for example, as illustrated and described below with respect to FIGS. 4-5. In some examples, the application software may be executed on an external device, for example, computing device 104 or distributed computing system 106, and may use information transferred from connectivity unit 210. In some examples, the application software may be compatible with one or more operating systems, e.g., Windows, iOS®, Android™, or any other suitable operating system.

In some examples, application software may include a mobile application, and may implement a Fast Fourier Transform (FFT) for frequency-domain processing of incoming information, e.g., executed by processing circuitry 126, 136, and/or 146. In some examples, FFT implementation in the mobile application may be executed and provide results within seconds. In some examples, FFT implementation may result in information such as frequency harmonic amplitudes and phase information, and may be used to derive immunoassay detection. In some implementations, harmonic amplitudes, phase angle information, and harmonic ratios may be metrics for quantifying target analytes from testing samples, such as sample vial 108.

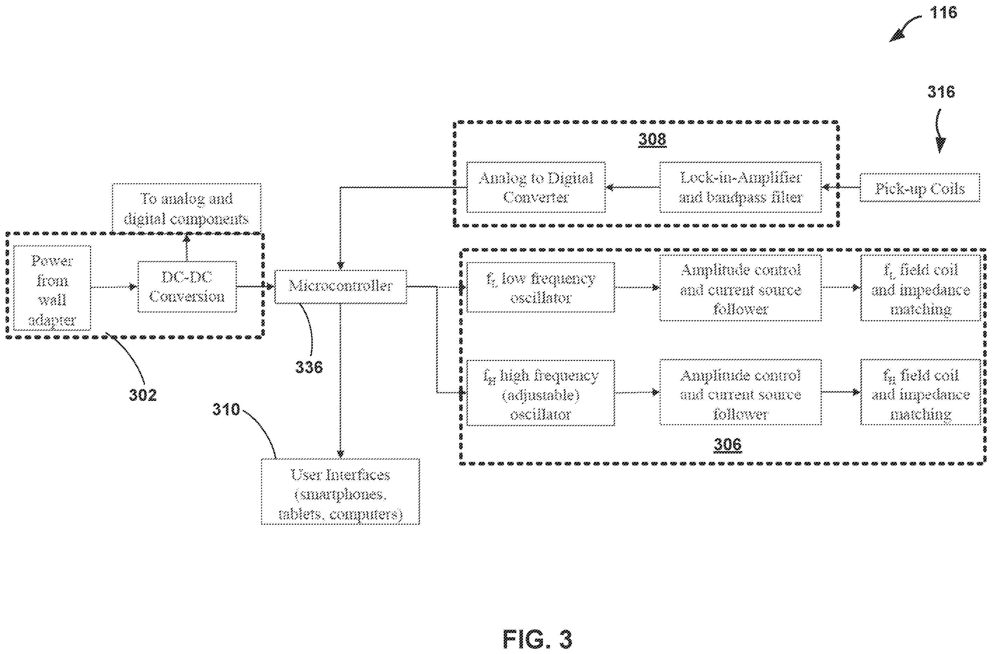

FIG. 3 is a block diagram of another example circuitry 116 of an example MPS handheld device 102, in accordance with one or more techniques of this disclosure. In the example shown, circuitry 116 includes power unit 302, microcontroller 336, coil driver 306, signal conditioning unit 308, connectivity unit 310, and coils 112.

In the examples shown, power unit 302 is configured to generate electrical power, for example, usable direct current (DC) voltages from an off-board alternating current (AC) power supply, e.g., a wall outlet. Power unit 302 may be substantially similar to power unit 202 illustrated and described above with respect to FIG. 2, and may additionally include DC to DC conversion.

Microcontroller 336 may be utilized as an on-board processor, e.g., as processing circuitry 136, and may be substantially similar to microcontroller 236 illustrated and described above with respect to FIG. 2.

In the example shown, coil driver 306 may be configured to generate variable frequency waveforms for drive coils, e.g. coils 112, and may be substantially similar to coil driver 206 illustrated and described above with respect to FIG. 2. In the examples shown, coil driver 306 may generate a low frequency waveform via a low frequency oscillator and a high frequency waveform via a high frequency oscillator. Coil driver 306 may be configured to control the amplitudes and impedance matching of the low and high frequency waveforms. In some examples, waveforms may include any of a sinusoidal waveform, a rectangular or square waveform, a triangular waveform, a sawtooth waveform, or any combination thereof.

In the example shown, signal conditioning unit 308 may be configured to remove noise and amplify a signal received from pick-up coils 316, and perform analog to digital conversion. In some examples, signal conditioning unit 308 may be substantially similar to signal conditioning unit 208. For example, one or more pick-up coils of coils 112 may generate a differential voltage output, and signal conditioning unit 308 may operate to condition the differential voltage signal similar to signal conditioning unit 208 illustrated and described above with respect to FIG. 2.

In the example shown, connectivity unit 310 may be configured to transfer data via a wired or wireless connection, e.g., connection 118. Connectivity unit 310 may be configured to provide an interface to external devices, e.g., external computing devices. In some examples, connectivity unit 310 may be substantially similar to connectivity unit 210 illustrated and described above with respect to FIG. 2.

FIGS. 4-5 are described in conjunction to describe an example method of measuring a sample using MPS handheld device 102.

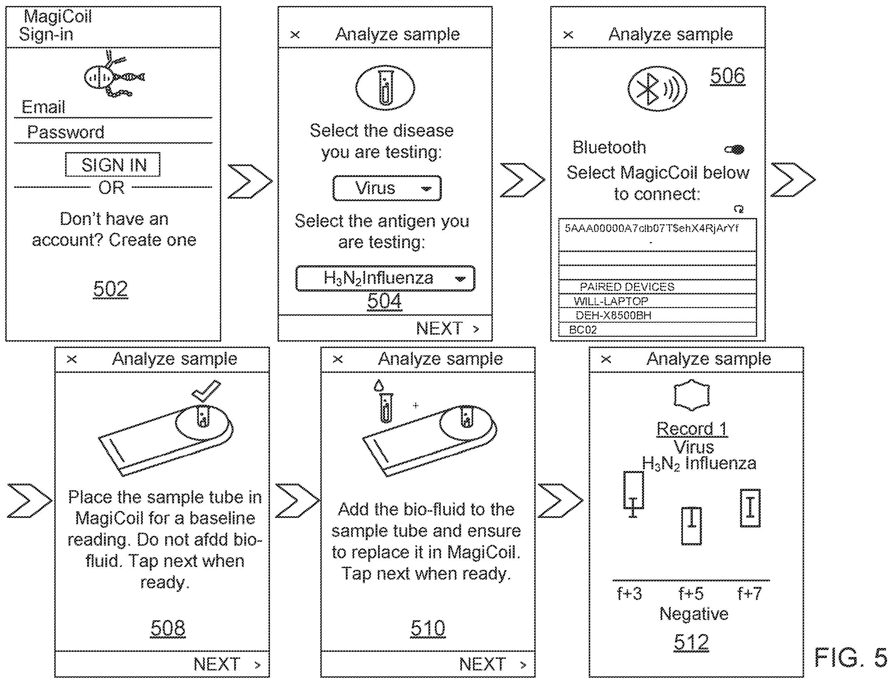

FIG. 4 is a flowchart of an example method 400 of measuring a sample using MPS handheld device 102, in accordance with one or more techniques of this disclosure. The example method is described with respect to the diagnosis system and circuitry 116 of FIGS. 1-3. The example method may be performed, for example, by a user interacting with an MPS handheld device and a computing device, such as MPS handheld device 102 and computing device 104 and/or distributed computing device 106, executing the steps of the method. FIG. 5 is an illustration of an example series of user interface screens 500 of a mobile application, in accordance with one or more techniques of this disclosure. In some examples, interface screens 500 may be used by a user to guide execution of method 400.

In the example shown in FIG. 5, a user may be prompted to enter login and password information as part of a secure login at screen 502. After secure login, the user may be prompted to select and disease to be tested and/or antigens to be tested at screen 504. At screen 506, the user may be prompted to select a device to establish communication with the computing device executing the application, for example, to connect with MPS handheld device 102.

A user may place a sample in MPS handheld device 102, e.g., a sample vial including surface functionalized MNPs without a biological sample added, and a baseline signal of the sample vial may be measured (402). Screen 508 may prompt the user to place the sample in MPS handheld device 102 and input a user input causing MPS handheld device 102 to measure the baseline signal (402).

Screen 510 may prompt the user to add the biological sample to the sample vial (404). In some examples, screen 510 may include a prompt indicating to the user to wait for a predetermined amount of time to allow mixing and/or chemical bonding of the biological sample and the surface functionalized MNPs.

Screen 510 may include a prompt instructing the user to perform a user input causing MPS handheld device 102 to measure a sample signal, and a processor may perform signal processing and diagnostics, and return results to the application. For example, screen 510 may prompt the user to tap a touch screen after waiting for the predetermined mixing and/or chemical bonding time. After the user performs the user input that causes MPS handheld device 102 to measure the sample signal, the user may wait for a predetermined time, during which time the surface functionalized MNPs may interact with magnetic fields generated by MPS handheld device 102, e.g., via drive coils, and generate a signal, e.g., via pick-up coils 316 as illustrated and described above with respect to FIG. 3. After the predetermined time, MPS handheld device 102 may measure the generated signal (408). After the measurement is complete, the application may display results at screen 512, for example, a positive or negative result for the disease and/or antigen selected at screen 504 and any other information.

In some examples, method 400 may be a wash-free, one-step bioassay method that may be performed by non-technicians with reduced and/or minimal training, and unbound target analytes may not need to be removed.

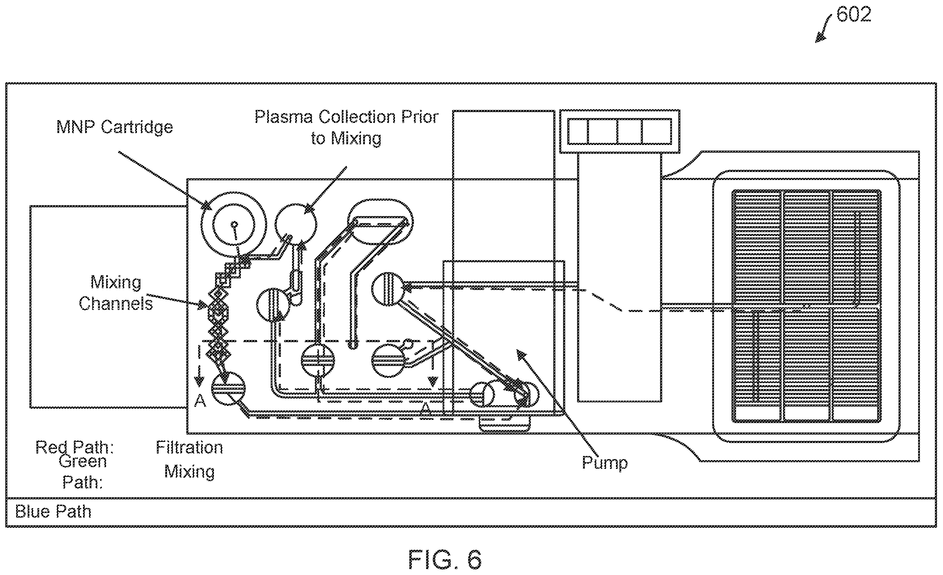

FIGS. 6-9 are diagrams illustrating various views of an example microfluidic device 602. FIG. 6 is a plan view of microfluidic device 602, in accordance with one or more techniques of this disclosure. In some examples, microfluidic device 602 may include functionality like MPS handheld device 102, in addition to the functionality described with reference to FIG. 6. In some examples, microfluidic device 602 may be configured to extract blood plasma from whole blood through filtration, then transfer the blood plasma to mix with MNPs, and to be sampled to detect presence of an analyte in the blood plasma. For example, the blood cells and other cells in the biofluid may potentially cause noise in MPS detection and filtering out the cells and macromolecular proteins may improve the sensitivity of MPS detection. In addition, microfluidic device 602 may be configured to effectively mix the blood plasma with MNP fluid and speed up the chemical binding process, e.g., the binding processes of antibody-antigen, aptamer-aptamer, aptamer-antigen, aptamer-ions, and the like, and may decrease the assay time. Further, microfluidic device 602 may be configured to use a smaller amount of MNPs and smaller coils.

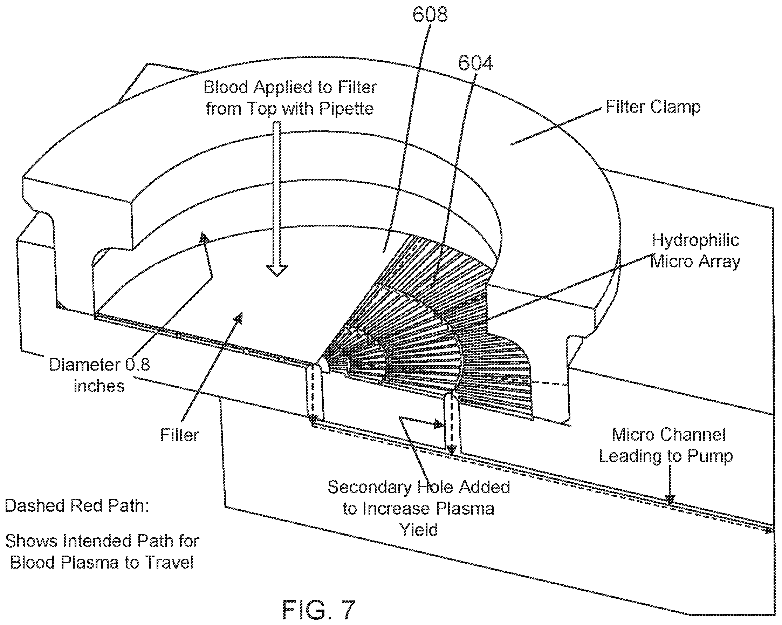

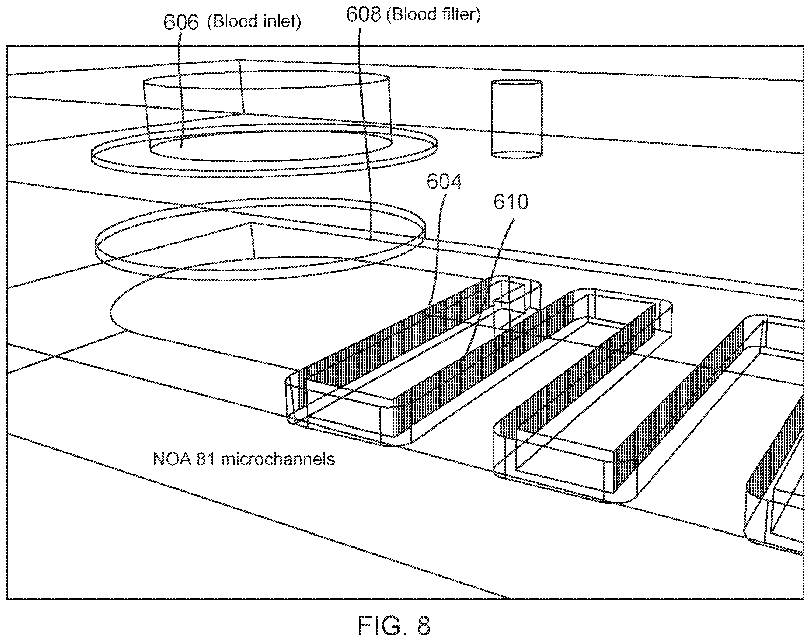

FIG. 7 is cross-sectional diagram of a portion of microfluidic device 602, in accordance with one or more techniques of this disclosure. FIG. 7 shows an example configuration of a filter device, which includes a filter and a hydrophilic micro array 604 that draws blood plasma through the filter. Hydrophilic microfluidic array 604 is configured to draw blood plasma into the channels 610 which route the plasma to one or more drains, as shown in FIG. 8, thereby causing at least a partial separation of blood plasma from white blood cells, red blood cells, platelets, and other cells.

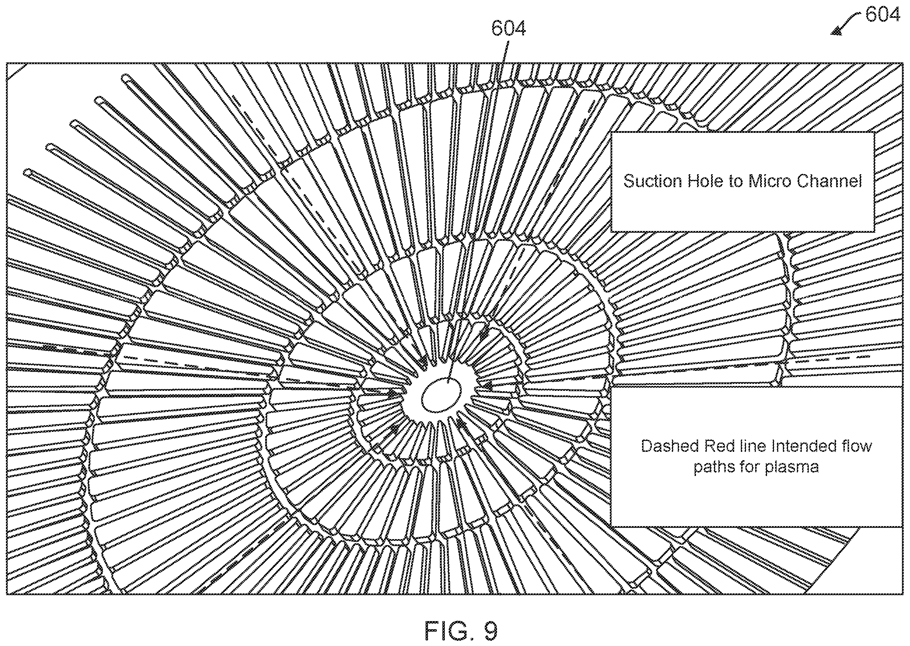

FIG. 9 is an illustration of example microfluidic channels 604 of an example microfluidic device 602, in accordance with one or more techniques of this disclosure. In the example shown, hydrophilic micro array 604 may be arranged in an array and include one or more drains for at least partially separated blood plasma.

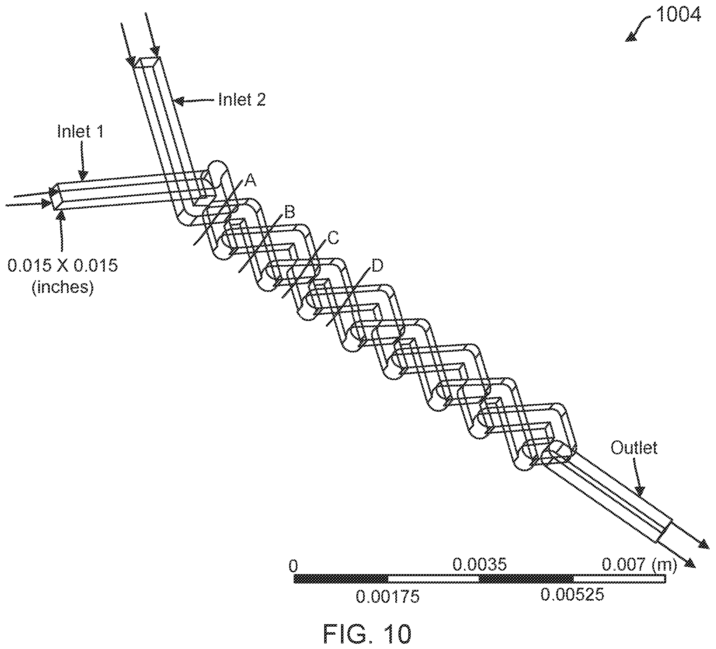

FIG. 10 is an illustration of example mixing microfluidic channels 1004 of microfluidic device 602, in accordance with one or more techniques of this disclosure. In the example shown, mixing microfluidic channels 1004 may be configured to mix a fluid including MNPs with a biofluid (e.g., blood plasma) sample. Microfluidic channels 1004 may be an example of the mixing channels shown in FIG. 6. For example, microfluidic channels 1004 may be disposed after microfluidic channels 610 and receive at least partially separated blood plasma from microfluidic channels 610 within microfluidic device 602 to mix with the fluid including MNPs. In some implementations, the inlets cross at vertical mixing intersections A-E.

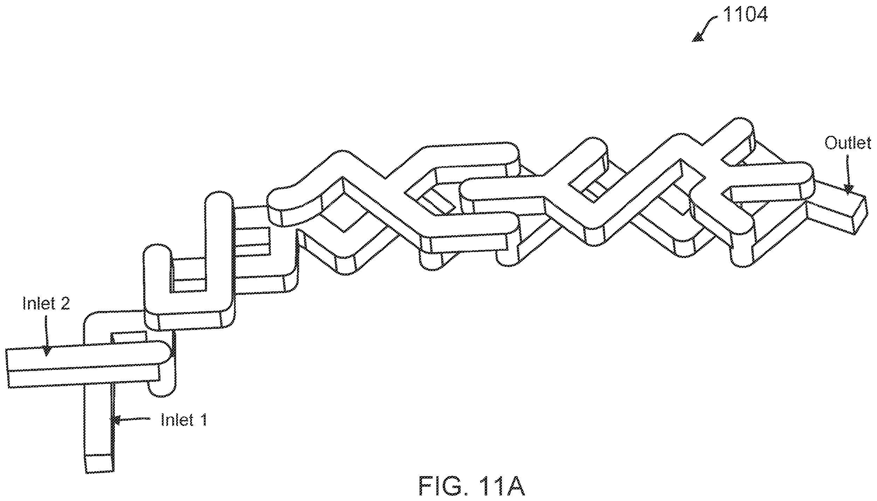

FIG. 11A is an illustration of another example of mixing microfluidic channels 1104 that may be used in microfluidic device 602, in accordance with one or more techniques of this disclosure. Mixing microfluidic channels 1104 may be an alternative mixing geometry as compared to mixing microfluidic channels 1004, and may be configured similarly to mixing microfluidic channels 1004 as described above with respect to FIG. 10.

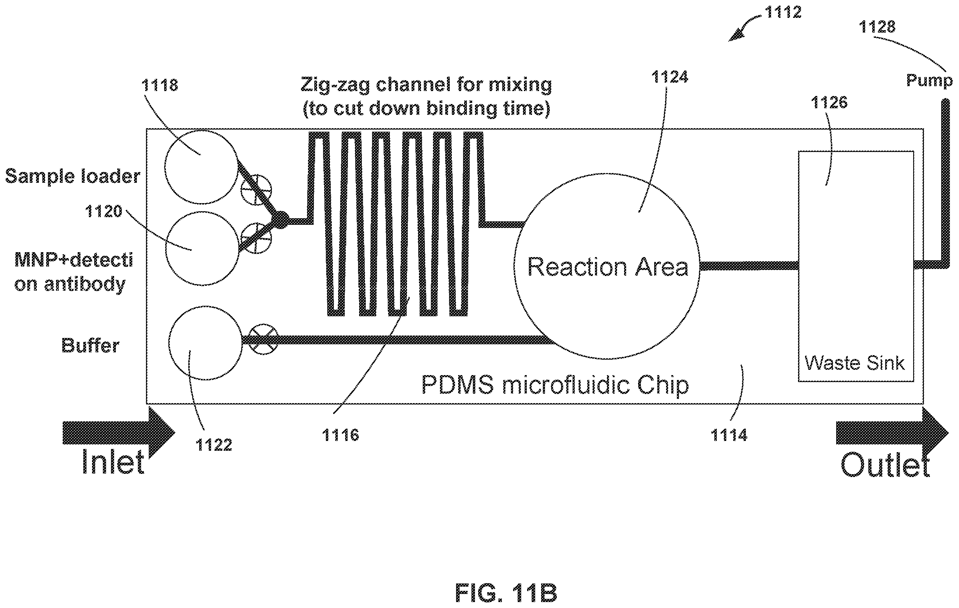

FIG. 11B is a block diagram of microfluidic device 1112, in accordance with one or more techniques of this disclosure. In some examples, microfluidic device 1112 may include functionality like MPS handheld device 102, in addition to the functionality described with reference to FIG. 11B. In some examples, microfluidic device 1112 may be configured to mix a sample with MNPs and detection antibodies, e.g., via mixing channels 1116. In the example shown, microfluidic device includes a polydimethylsiloxane (PDMS) microfluidic chip 1114 including a sample area 1118, an MNP area 1120, a buffer area 1122, microfluidic channels 1116, a reaction area 1124, a waste area 1126, and a pump 1128. In some examples, microfluidic device 1112 may be configured to be used, e.g., as an add-on mixing device, with any of the MPS devices described herein.

In the example shown, sample area 1118 and MNP area 1120 are fluidically connected to each other and microfluidic channels 1116. Sample area 1118 may be configured to receive biological samples, and provide biological samples to microfluidic channels 1116, e.g., via valve 3. MNP area 1120 may be configured to receive surface functionalized MNPs and provide surface functionalized MNPs to microfluidic channels 116, e.g., via valve 2. Microfluidic channels 1116 may be configured to mix the biological samples and surface functionalized MNPs, e.g., for MPS detection techniques described herein.

Buffer area 1122 may be fluidically connected to reaction area 1124, e.g., via valve 1 and may be configured to receive a solution configured to mix with MNPs and biological samples, e.g., to achieve a desired dilution. Microfluidic channels 1116 may be fluidically coupled to reaction area 1124, and pump 1128 may be configured to move fluids from sample area 1118, MNP area 1120, and buffer 1122 to reaction area 1124 and from reaction area 1124 to waste sink 1126.

FIGS. 12-14 illustrate one or more example operating principles of an MPS device, for example, MPS handheld device 102, and will be described concurrently below.

FIG. 12A is a schematic cross-sectional illustration of a portion of an example MPS handheld device 102, in accordance with one or more techniques of this disclosure. In the example shown, sample vial 108 is in position within sample loading port 110 in MPS handheld device 102 during a measurement, e.g., while drive coils 1212 and 1214 generate an alternating magnetic field H(t) proximate sample vial 108 and pick-up coils 1216 detect the resulting magnetic responses from MNPs within sample vial 108. Pick-up coils 1216 may be designed to have half of its portion clockwise wound and the other half portion counter-clockwise wound. This design removes the signal caused by alternating magnetic field H(t) and allows the pick-up coils 1216 to specifically detect the magnetic responses of MNPs. In some examples, drive coils 1212 may generate a magnetic field having a low frequency, $f_L$, relative to a magnetic field generated by drive coils 1214 having a higher frequency, $f_H$ based on drive signals generated by processing circuitry of MPS handheld device 102. Each of drive coils 1212 and 1214 may additionally generate magnetic fields having different amplitudes, e.g., $A_L$ generated via low frequency drive coils 1212 and $A_H$ generated via high frequency drive coils 1214. The magnetic fields generated by drive coils 1212 and 1214 may be in phase, and may add via superposition, resulting in generation of the composite magnetic field H(t). In some examples, drive coils 1212 may be wound in an opposite direction from drive coils 1214, e.g., counter-clockwise for drive coils 1212 and clockwise for drive coils 1214, or clockwise for drive coils 1212 and counter-clockwise for drive coils 1214. In some examples, drive coils 1212 and 1214 may be wound in the same direction.

FIG. 12B is an illustration of an example plot 1220 of the amplitude of composite magnetic field H(t) as a function of frequency, in accordance with one or more techniques of this disclosure. In the example shown, drive coils 1212 and 1214 each generate a sinusoidal magnetic field, and plot 1220 illustrates the frequency content of composite magnetic field H(t), namely two substantially single frequency spikes, or delta functions, at $f_L$ and $f_H$ and having amplitudes $A_L$ and $A_H$, respectively.

FIG. 12C is an illustration of an example plot 1250 of the amplitude of magnetic flux B(t) due to the magnetic responses of MNPs detected by pick-up coils 1216 as a function of frequency, in accordance with one or more techniques of this disclosure. In the example shown, the MNPs within sample vial 108 respond to magnetic field H(t), which may generate harmonics of H(t) that are detected by pick-up coils 1216. In the example shown, the magnetic flux B(t) including magnetic responses of MNPs may include several harmonic frequencies of varying amplitudes at various frequencies.

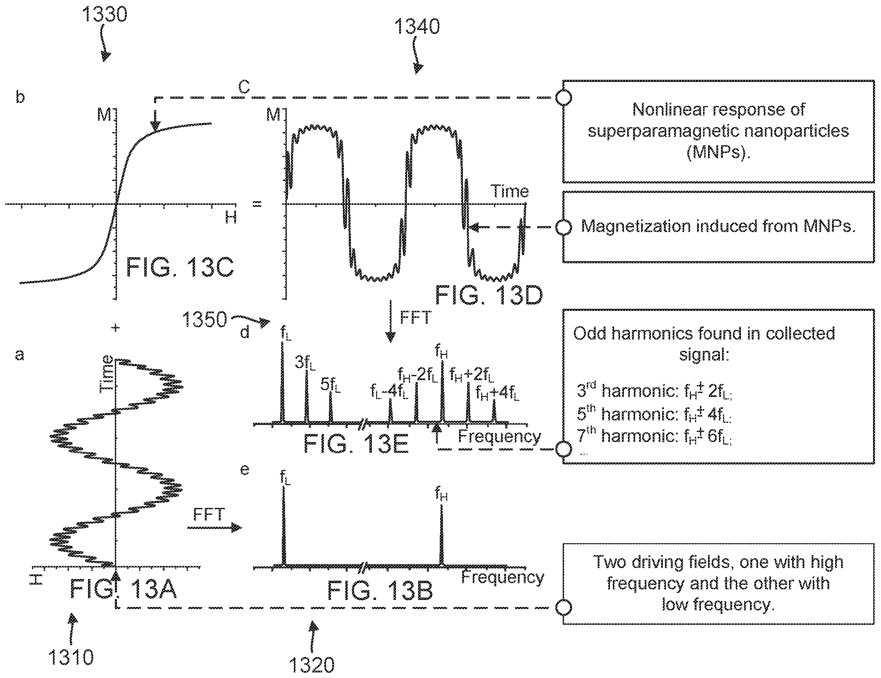

FIGS. 13A-13E illustrate the operating principle of FIGS. 12A-12C in more detail.

FIG. 13A is an illustration an example plot 1310 of the amplitude of an alternating magnetic field H(t) generated by drive coils as a function of time, in accordance with one or more techniques of this disclosure. For example, drive coils 1212 and 1214 may generate sinusoidal magnetic fields, and FIG. 13A illustrates the superposition of each low and high frequency sinusoidal magnetic field generated by drive coils 1212 and 1214, along with their corresponding differing amplitudes. FIG. 13B is an illustration an example plot 1320 of the amplitude of composite magnetic field H(t) as a function of frequency, in accordance with one or more techniques of this disclosure. In the example shown, FIG. 13B illustrates the spectral (temporal) content of H(t) of FIG. 13A, similar to FIG. 12B described above.

FIG. 13C is an illustration of an example plot 1330 of the magnetic response of MNPs to static magnetic fields (not alternating magnetic field), in accordance with one or more techniques of this disclosure. In the example shown, MNPs may by superparamagnetic, and may have a nonlinear response to an applied magnetic field.

FIG. 13E is an illustration of an example plot 1350 of the amplitude of the alternating magnetic responses of FIG. 13D as a function of frequency, in accordance with one or more techniques of this disclosure. FIG. 13D explicitly illustrates the additional harmonic content generated by the magnetic response of MNPs within sample vial 108.

In some examples, the magnetic response of MNPs within sample vial 108 may change based on the presence of analytes in a biofluid added to sample vial 108. For example, the analytes may bind to the surface functionalized MNPs and alter the magnetic response of the MNPs relative to the magnetic response of surface functionalized MNPs with no analytes present in the biofluid. In some examples, the altered magnetic response of the MNPs due to analytes may be distinguished from features of detected magnetic flux B(t), for example, via changes to the amplitudes and/or frequencies of the spectral content, e.g., harmonics, of B(t).

For example, in the presence of oscillating magnetic fields, MNPs may be magnetized and their magnetic moments may tend to align with the magnetic fields. For a ferrofluid system of monodispersed, noninteracting MNPs, the magnetic response may obey a Langevin function:

$$M_D(t) = m_s c L(\xi),$$

where,

$$L(\xi) = \coth \xi - \frac{1}{\xi}, \xi = \frac{m_s H(t)}{k_B T}$$

The MNPs are characterized by magnetic core diameter D, saturation magnetization $M_S$ and concentration $c$. In some examples, MNPs may be assumed to be spherical and without mutual interactions. Consequently, the magnetic moment of each particle may be $m_s = M_S \pi D^3 / 6$, where $V_c = \pi D^3 / 6$ is the volume of the magnetic core, $\xi$ is the ratio of magnetic energy over thermal energy, $k_B$ is Boltzmann's constant, and $T$ is the absolute temperature in Kelvin. The external magnetic fields may be expressed as $H(t) = A_H \cos(2\pi f_H t) + A_L \cos(2\pi f_L t)$ where $A_H$, $A_L$, $f_H$, and $f_L$ are the amplitude and frequency of high and low frequency fields, respectively.

The harmonics generated by MNPs at specific frequencies may be represented by a phasor, e.g., $Ae^{j(\omega t + \varphi)}$, or $A < \varphi$, where $\omega$ is the angular frequency of the driving field, A is the harmonic amplitude, $\varphi$ is the harmonic phase, and j is the square root of negative one.

FIG. 13D is an illustration of an example plot 1340 of the amplitude of an alternating magnetic responses of MNPs detected by pick-up coils 1216 as a function of time, in accordance with one or more techniques of this disclosure. The example shown in FIG. 13D illustrates the effects of the MNPs on the applied magnetic field, e.g., perturbation of H(t) resulting in generation of harmonics.

According to Faraday's law, the induced voltage in a pair of pick-up coils is expressed as:

$$u(t) = -S_0 V \frac{d}{dt} M_D(t)$$

where V is the volume of an MNP suspension. Pick-up coil sensitivity $S_0$ is equal to the external magnetic field strength divided by current.

Taylor expansion of $M_D(t)$ shows the major frequency mixing components:

$$\frac{M_D(t)}{m_s c} = L\left(\frac{m_s H(t)}{k_B T}\right)$$

$$= \frac{1}{3}\left(\frac{m_s}{k_B T}\right) H(t) - \frac{1}{45}\left(\frac{m_s}{k_B T}\right)^3 H(t)^3 + \frac{2}{945}\left(\frac{m_s}{k_B T}\right)^5 H(t)^5 + \cdots$$

$$= \cdots + \left[-\frac{1}{60} A_H A_L^2 \left(\frac{m_s}{k_B T}\right)^3 + \cdots\right] \times \cos\left[2\pi(f_H \pm 2f_L)t + \varphi_{f_H \pm 2f_L}\right]$$

$$+ \left[\frac{1}{1512} A_H A_L^4 \left(\frac{m_s}{k_B T}\right)^5 + \cdots\right] \times \cos\left[2\pi(f_H \pm 4f_L)t + \varphi_{f_H \pm 4f_L}\right]$$

$$+ \cdots$$

The mixing frequency components are found at odd harmonics exclusively:

$$M_D(t)\Big|_{3rd} \approx \frac{-m_s c}{60} A_H A_L^2 \left(\frac{m_s}{k_B T}\right)^3 \times \cos\left[2\pi(f_H + 2f_L)t + \varphi_{f_H \pm 2f_L}\right]$$

$$M_D(t)\Big|_{5th} \approx \frac{m_s c}{1512} A_H A_L^4 \left(\frac{m_s}{k_B T}\right)^5 \times \cos\left[2\pi(f_H + 4f_L)t + \varphi_{f_H \pm 4f_L}\right]$$

Amplitudes of induced voltages at the 3rd and 5th harmonics may be expressed as:

$$u_{3rd} = -S_0 V \frac{d}{dt} M_D(t)\Big|_{3rd}$$

$$u_{5th} = -S_0 V \frac{d}{dt} M_D(t)\Big|_{5th}$$

The harmonic amplitudes of the 3$^{rd}$ and 5$^{th}$ harmonics may be simplified as:

$$A_{3rd} \propto (f_H \pm 2f_L) \times cos\left[\varphi_{f_H \pm 2f_L}\right]$$

$$A_{5th} \propto (f_H \pm 4f_L) \times cos\left[\varphi_{f_H \pm 4f_L}\right]$$

where

$$\varphi_{f_H \pm 2f_L} = \arctan(2\pi(f_H \pm 2f_L)\tau_{eff})$$

For iron oxide MNPs with diameters of 20 nanometers (nm), the effective relaxation time is dominated by Brownian relaxation:

$$\tau = \tau_{eff} = \tau_{B0} = \frac{3\eta V_h}{k_B T}$$

In some examples, a change in MNP hydrodynamic size may cause a change in harmonic angle (phase angle), which further may cause a change in harmonic amplitude. Harmonic amplitudes may be proportional to the number of MNPs in a testing vial, and to make each testing result repeatable, a harmonic ratio of the 3$^{rd}$ harmonic over the 5$^{th}$ harmonic may be used to reduce and/or eliminated the effect of MNP quantities in the testing vial. The harmonic ratio of the 3$^{rd}$ over the 5$^{th}$ harmonics may be expressed as:

$$R35 = \frac{u_{3rd}}{u_{5th}} = \frac{\frac{d}{dt} M_D(t)|_{3rd}}{\frac{d}{dt} M_D(t)|_{5th}} = \frac{(f_H \pm 2f_L) \times cos\left[\varphi_{f_H \pm 2f_L}\right]}{(f_H \pm 4f_L) \times cos\left[\varphi_{f_H \pm 4f_L}\right]}$$

In some examples, because $f_L \ll f_H$, the harmonic ratio of the 3$^{rd}$ over the 5$^{th}$ may be further simplified as:

$$R35 = \frac{cos\left[\varphi_{f_H \pm 2f_L}\right]}{cos\left[\varphi_{f_H \pm 4f_L}\right]}$$

In some examples, a change in MNP hydrodynamic size may cause a change in harmonic angle, which may further cause a change in the harmonic amplitude ratio.

Harmonic ratio may be used as an MNP quantity-independent parameter to monitor the binding of target analytes onto MNPs, e.g., the hydrodynamic size change. In addition, any kinds of harmonic ratios such as R37 (the 3$^{rd}$ over the 7th harmonic ratio), R57 (the 5th over the 7$^{th}$ harmonic ratio), Rij (the ith over the jth harmonic ratio, where i and j are odd numbers and i≠j), or any other harmonic ration, may be used.

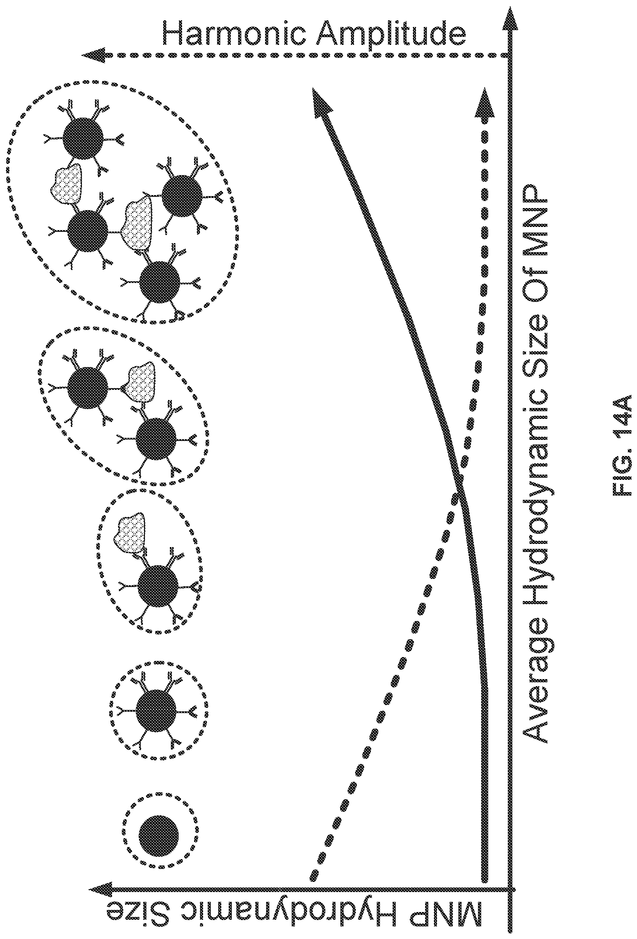

FIG. 14A is an illustration of an example plot of the harmonic amplitude response as a function of hydrodynamic size of MNPs, in accordance with one or more techniques of this disclosure. In the example shown, as the degree of MNP self-assembly increases in the presence of one or more analytes, the average hydrodynamic size of MNPs and MNP self-assemblies, e.g., clusters, increases, and the measured harmonic amplitudes decrease.

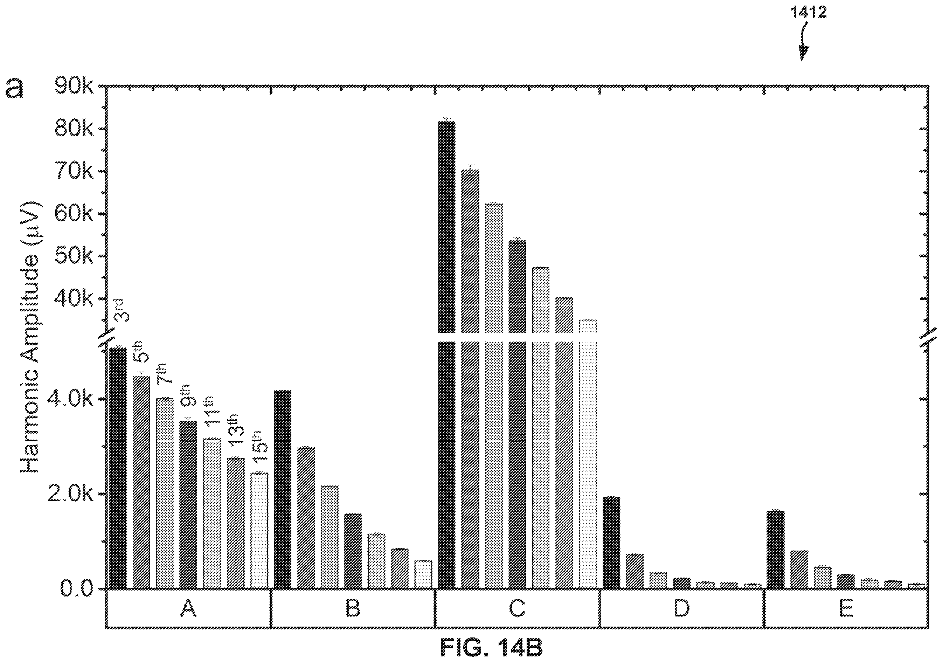

FIG. 14B is an illustration of an example plot 1412 of the harmonic amplitudes of different types of MNPs, in accordance with one or more techniques of this disclosure. The example of FIG. 14B illustrates harmonic amplitudes that may be indicative of an MNP type and/or sample having the relatively largest MPS harmonic amplitude per weight of iron oxide MNP, e.g., for detectability by an MPS device such as MPS handheld device 102 of FIG. 1. In the example shown, the harmonic amplitudes (e.g., referred to as "harmonics" hereinafter) are arranged in ascending harmonic order, e.g., 3rd, 5th, 7th, 9th, 11th, 13th, and 15th, for each MNP type A-E. In the example shown, the five iron oxide MNP types corresponding to A-E in FIG. 14B and in Table 1 below include SHB30 and SHP30 from Ocean Nano Tech LLC, MP25 BN and MP25 from Nanocs Inc.

Table 1

| sample index | concentration (mg/mL) | surface | $H_c$ (Oe)[a] | $M_s$ (@ 2000 Oe)[a,b] | M (@ 300 Oe)[a,c] | A3 ($\mu$V)[d] | A3/$\mu$g MNP ($\mu$V/$\mu$g)[e] |
|---|---|---|---|---|---|---|---|
| A: SHB30 | 0.8 | biotin | ±24.5 | 68.3 emu/g | 59.3 emu/g | 5067 | 79.2 |
| | | | | 54.6 $\mu$emu/$\mu$L | 47.4 $\mu$emu/$\mu$L | | |
| B: SHB30 | 3 | biotin | ±74.1 | 33.5 emu/g | 14.8 emu/g | 4169 | 17.4 |
| | | | | 100.5 $\mu$emu/$\mu$L | 44.4 $\mu$emu/$\mu$L | | |
| C: SHP30 | 17.5 | carboxylic | ±22.4 | 63.1 emu/g | 49.2 emu/g | 81,630 | 58.3 |
| | | | | 1104.3 $\mu$emu/$\mu$L | 861 $\mu$emu/$\mu$L. | | |
| D: MP25 BN | 1-2 | biotin | ±13.4 | 26.3-52.7 emu/g | 14.7-29.4 emu/g | 1924 | 12.0-24.0 |
| | | | | 26.3-105.4 $\mu$emu/$\mu$L | 14.7-58.8 $\mu$emu/$\mu$l | | |
| E: MP25 CA | 2 | carboxylic | ±15.9 | 17.6 emu/g | 9.5 emu/g | 1639 | 10.2 |
| | | | | 35.2 $\mu$emu/$\mu$L | 19 $\mu$emu/$\mu$L | | |

In the example shown, the five MNPs A-E include varying sizes, concentrations, magnetic properties, and surface modifications. Samples A-C are iron oxide nanoparticles with an average magnetic core size of 30 nm. Samples D and E are iron oxide nanoparticles with an average magnetic core size of 25 nm. MNPs from samples A, B, and D are coated with biotin, while MNPs from samples C and E are coated with a carboxylic group. These different surface coating layers act as reactive groups and improve the colloidal stability of MNP suspensions. As a result, the coating layers increase the hydrodynamic size of MNPs by ~10 nm. Eighty microliters of MNP suspension is drawn to a glass vial for MPS measurements, e.g., via MPS handheld device 102. Each of samples A-E may be additionally measured via standard direct current (DC) hysteresis loop and the saturation magnetization of the MNPs, Ms (unit: emu/g), may be ranked. In the example shown, samples A-E may be ranked as: A > C > B ~ D > E. Due to the varying concentrations of MNPs in each sample, the magnetic moment per volume of suspension is calculated and listed in Table 1. Although all MNPs of samples, A-E are iron oxide nanoparticles, their saturation magnetizations are different due to different ratios of $\gamma$-Fe$_2$O$_3$, Fe$_3$O$_4$, and $\alpha$-Fe$_2$O$_3$ materials used for synthesizing the magnetic cores. Sample C, with the highest MNP concentration, yields the highest magnetic moment per microliter volume (unit: $\mu$emu/$\mu$L). The magnetic moment per volume of MNPs from highest to lowest are ranked as: C > A > B ~ D > E. All the MNP samples show negligible coercivities.

In the example shown in FIG. 14B, the harmonic amplitudes from highest to lowest may be ranked as: C > A > B > D > E, which agrees with the DC hysteresis loop measurements of magnetic moment per volume of MNP suspension. In addition, these higher harmonics may be caused by nonlinear magnetic responses of MNPs under AC magnetic fields. Thus, the degree of nonlinearity in the hysteresis loops under an external magnetic field of -300 oersted (Oe) to +300 Oe may cause the higher harmonic amplitudes in samples A and C and lower harmonic amplitudes in samples D and E. Since the hydrodynamic sizes of MNPs from samples A-E are similar, the MNP amount/concentration and the nonlinearity of magnetization curves directly cause the different magnetic responses and different magnitudes of higher harmonics. As illustrated in table 1 above, sample A has the largest MPS harmonic amplitude per microgram of iron oxide MNP, and may therefore allow for bioassays with a minimum and/or reduced amount of MNPs per test.

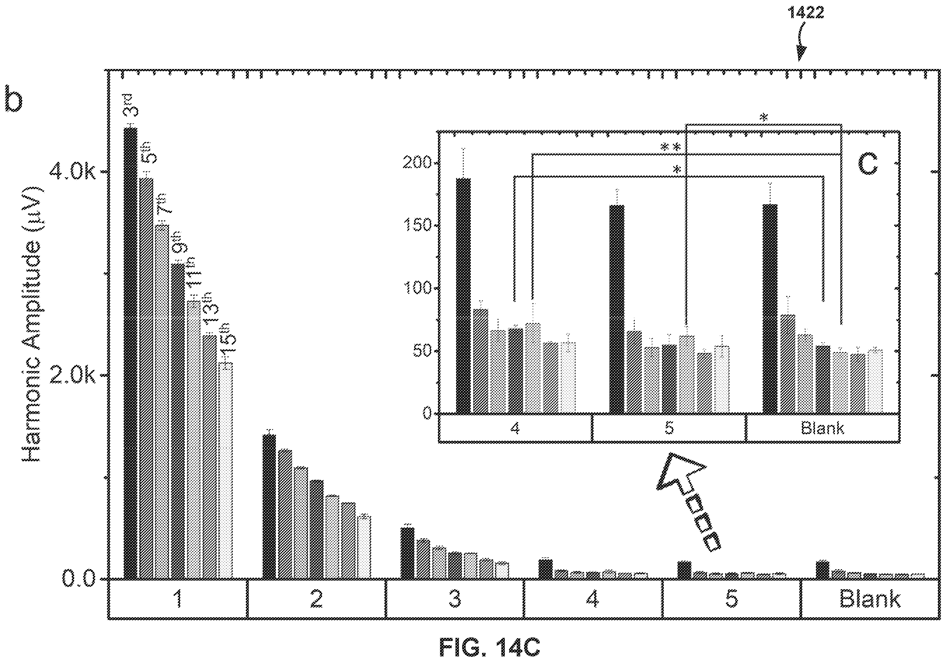

FIG. 14C is an illustration of an example plot 1422 of the harmonic amplitudes of different amounts of a type of MNP, in accordance with one or more techniques of this disclosure. The example of FIG. 14C illustrates harmonic

13

amplitudes that may be indicative of a minimum amount of MNPs of a particular MNP type, e.g., for detectability by an MPS device such as MPS handheld device 102 of FIG. 1. In the example shown, harmonics of measurements of five concentrations (samples 1-5) of the SHB30 (sample A of FIG. 14B) and of a zero concentration (Blank) are shown.

In the example shown, the SHB30 MNPs are prepared by two-fold dilutions such that the MNP weight is 64 $\mu$g for sample 1, 32 $\mu$g for sample 2, 16 $\mu$g for sample 3, 8 $\mu$g for sample 4, and 4 $\mu$g for sample 5. The Blank sample is 80 $\mu$L of phosphate-buffered saline (PBS). Sample 1 of plot 1422 has highest MPS signals followed by samples 2 and 3, and although samples 4 and 5 show similar harmonic amplitudes compared to the Blank sample, a two-sample t test results show that the $9^{th}$ and the $11^{th}$ harmonics from sample 4 are statistically significantly different from the Blank sample, (e.g., with p-values of 0.034 and 0.01, respectively). In addition, the $11^{th}$ harmonic from sample 5 is significantly different from the Blank sample, with a p-value of 0.04 for the example shown. In the example, the MPS device, e.g., MPS handheld portable device 102, may therefore detect as low as 4 $\mu$g of iron oxide MNPs, and a calculated lowest detectable magnetic moment by this portable device is 273.2 $\mu$emu, e.g., a signal from the Blank sample.

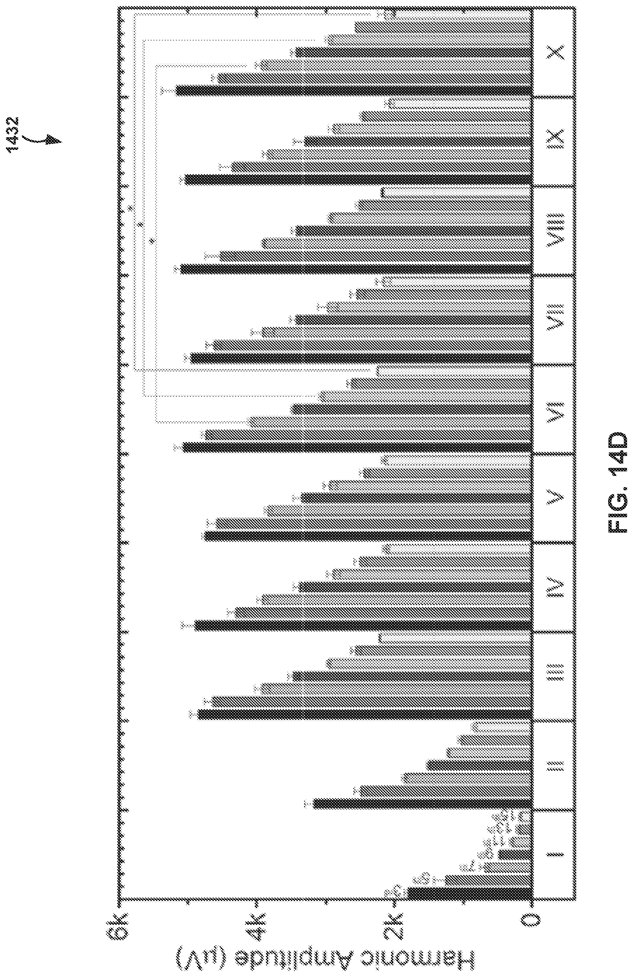

FIG. 14D is an illustration of an example plot 1432 of the harmonic amplitudes of an MPS measurement of streptavidin, in accordance with one or more techniques of this disclosure. The example of FIG. 14D illustrates harmonic amplitudes that may be indicative of a minimum amount of streptavidin, e.g., an example of an analyte detectability of an MPS device such as MPS handheld device 102 of FIG. 1. In the example shown, harmonics of measurements of ten amounts of streptavidin I-X are shown. The harmonics illustrated in plot 1432 may be measured using the SHB30 MNPs, e.g., sample A of FIG. 14B, because those MNPs have the highest MPS harmonic amplitude per microgram of iron oxide, allowing for a reduced and/or minimum amount of MNPs to be used per test/measurement, relative to samples B-E.

In the example shown, 80 $\mu$L of 1 mg/mL SHB30 iron oxide MNPs that are surface functionalized with biotin are mixed with varying concentrations/amounts of 80 $\mu$L of streptavidin protein. For example, samples I-IX are prepared by twofold dilution of streptavidin, such that the streptavidin concentrations of samples I-IX, respectively in order from I to IX, are 2048 nM, 1024 nM, 512 nM, 256 nM, 128 nM, 64 nM, 32 nM, 16 nM, and 8 nM. Sample X is prepared by mixing 80 $\mu$L of 1 mg/mL SHB30 iron oxide MNPs with 80 $\mu$L of PBS. The corresponding MNP to streptavidin ratios of samples I-IX, respectively in order from I to IX, are 1:60, 1:30, 1:15, 1:7.5, 1:3.75, 1:1.88, 1:0.94, 1:0.47, and 1:0.24. All of samples I-X may be incubated at room temperature for 30 minutes to allow the binding of streptavidin molecules to biotins from the MNP surface.

Each bar of plot 1432 represents an average harmonic amplitude measurement of multiple measurements of each sample I - X, e.g., six measurements in the example shown. A two-sample t test may be performed on each sample to compare with the control, e.g., sample index X with a 0 nM streptavidin concentration. In the example shown, the 3rd harmonic amplitudes from samples I-V are significantly different from the control sample X with p-values smaller than 0.05. The 3rd harmonics from samples VI-IX are not significantly different from the control sample X with p-values larger than 0.05. As such, for the example of FIG. 14D, the detection limit of the MPS device used (e.g., MPS handheld device 102) for streptavidin is 128 nM (equal to 10.24 pmole) based on the $3^{rd}$ harmonics.

The larger amount of streptavidin proteins of samples I - II may cause the MNPs of those samples to form clusters. Due to the cross-linking of MNPs and streptavidin, MNPs may be closely compacted and their hydrodynamic sizes may dramatically increase. As a result, the Brownian relaxation of bound MNPs may be blocked and their dynamic magnetic responses may become weaker. In the example shown, because of the larger amount of streptavidin in samples I and II, their respective harmonic amplitudes are significantly lower than samples, III - X, e.g., due to the larger hydrodynamic sizes of the MNPs of those samples.

In some examples, one or more higher harmonics, alone or in conjunction with the $3^{rd}$ harmonics may be used and may improve the detection limit of MPS handheld device 102. For example, the 7th, the 11th, and the 15th harmonics of sample VI are significantly different from control sample X, e.g., with two-sample t test p-values smaller than 0.05, indicating that MPS handheld device 102 may detect as low as 64 nM of streptavidin (equal to 5.12 pmole). In some examples, MPS handheld device 102 measurement of target biomarkers may be based on any MPS higher order harmonics, e.g., $3^{rd}$ through $15^{th}$ harmonics of plot 1432 or any other harmonics, alone or in any combination. In some examples, MPS handheld device 102 measurement of target biomarkers may alternatively or additionally be based on higher order phase angles and harmonic ratios, and which may be MNP quantity independent.

In some examples, e.g., as with target analytes from complex biological media such as blood, nasal swab, sputum sample and the like, further surface modifications of the MNPs may be made, e.g., to minimize and/or block nonspecific bindings (NSBs). In some examples, block buffers and/or blocking reagents such as dextrans, ethanolamine, bovine serum albumin (BSA) and the like, may be used after MNP surface functionalization, e.g., to occupy remaining NSB sites.

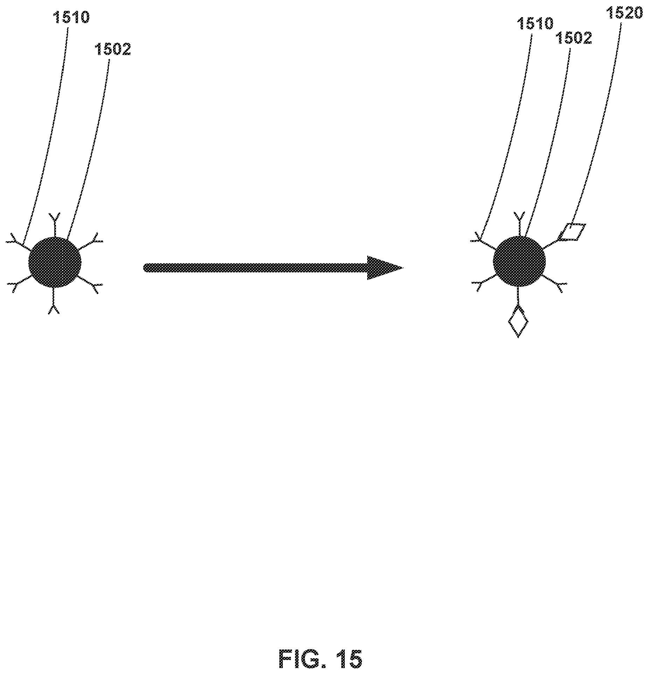

FIGS. 15-24 illustrate example chemical and mechanical processes that may cause MNP hydrodynamic size to change in the presence of one or more analytes, and may be detectable via MSP, for example, by using MSP handheld device 102. In some examples, the example chemical and mechanical processes causing MNP hydrodynamic size change may be distinguishable via harmonic amplitude and harmonic amplitude ratio changes.

FIG. 15 is an illustration of an example surface functionalized MNP 1502, in accordance with one or more techniques of this disclosure. In the example shown, MNP 1502 may be surface functionalized via a coating of one or more monoclonal antibodies 1510. Monoclonal antibodies 1510 may be selected to bind to a single antigen or antibody. In some examples, one or more antigens 1520 may bind to the one or more monoclonal antibodies 1510, for example, via addition of a biofluid containing antigens 1520 to sample vial 108 including MNPs 1502. In some examples, antigens 1520 may be one or more analytes, for example, antigens 1520 may be one or more analytes of a coronavirus such as human coronavirus 229E, human coronavirus OC43, SARS-CoV (2003), HCoV NL63 (2004), HKU1 (2005), MERS-CoV (2102), SARS-CoV-2, and the like. In some examples, antigens 1520 may be any of a membrane, an envelope, a structure protein, a Hemagglutinin esterase protean, a nucleocapsid protein inside the envelop, and the like, of a coronavirus. MNP 1502 may have an increased hydrodynamic size as a result of the binding of antigens 1520 to monoclonal antibodies 1510. The increased hydrodynamic size may change the relaxation time of MNP 1502 in response to the applied magnetic fields described herein, which may affect the magnetic fields detected by the pick-up coil.

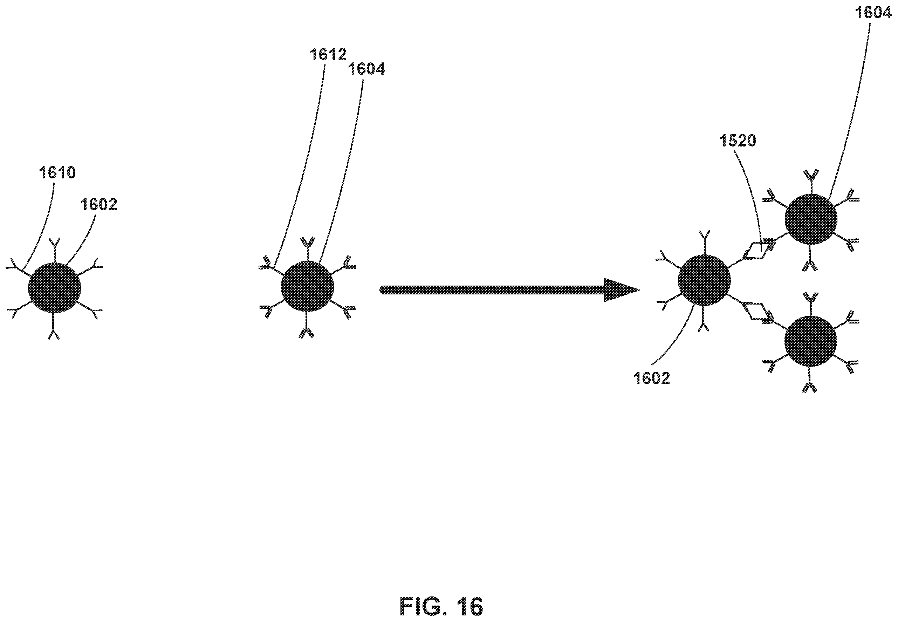

FIG. 16 is an illustration of example surface functionalized MNPs 1602 and 1604, in accordance with one or more techniques of this disclosure. In the example shown, MNP 1602 may be surface functionalized via a coating of one or more antibodies 1610, and MNP 1604 may be surface functionalized via a coating of one or more antibodies 1612, where antibodies 1610 may be different from antibodies 1612. For example, antibodies 1610 may be configured to bind to a first portion of an antigen 1520 and antibodies 1612 may be configured to bind to a second portion of the antigen 1520. In some examples, one or more antigens 1520 may bind to either of the one or more antibodies 1610 and 1612, for example, via addition of a biofluid containing antigens 1520 to sample vial 108 including MNPs 1602 and 1604. In the example shown, MNPs 1602 and 1604 may cluster as a result of binding with antigens 1520, and in some examples, MNPs 1602 and 1604 may have an increased hydrodynamic size as a result of the binding of antigens 1520 to antibodies 1610 and 1612. The configuration of FIG. 16 may be more sensitive than the configuration of FIG. 15, as the hydrodynamic size of particles changes to a greater extent in response to binding of an antigen 1520 to antibodies 1610 and 1612. In some examples, antigen 1520 may be substantially similar to antigen 1520 illustrated and described above with respect to FIG. 15.

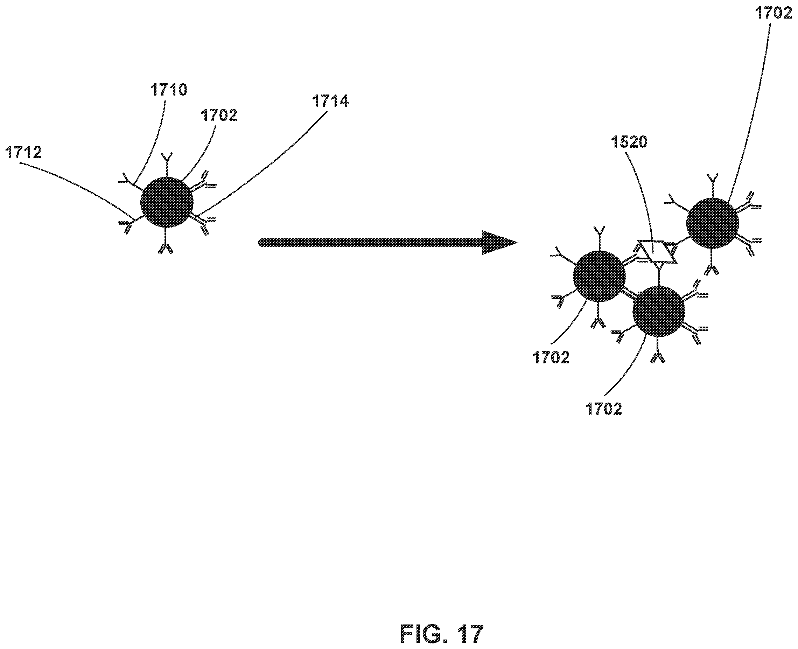

FIG. 17 is an illustration of an example surface functionalized MNP 1702, in accordance with one or more techniques of this disclosure. In the example shown, MNP 1702 may be surface functionalized via a coating of one or more polyclonal antibodies 1710, 1712, and 1714, where polyclonal antibodies 1710, 1712, and 1714 may be different from each other. In some examples, MNP 1702 may be coated with fewer or more than three polyclonal antibodies as illustrated in FIG. 17. In some examples, one or more antigens 1520 may bind to any of the one or more polyclonal antibodies 1710, 1712, and 1714, for example, via addition of a biofluid containing antigens 1520 to sample vial 108 including MNPs 1702. For example, each of polyclonal antibodies 1710, 1712, and 1714 may be configured to bind to different binding sites of antigen 1520. In the example shown, a plurality of MNPs 1702 may cluster as a result of binding with antigen(s) 1520, and in some examples, MNPs 1702 may have an increased hydrodynamic size as a result of the binding of antigens 1520 to antibodies 1710, 1712, and/or 1714. In some examples, antigen 1520 may be substantially similar to antigen 1520 illustrated and described above with respect to FIG. 15.

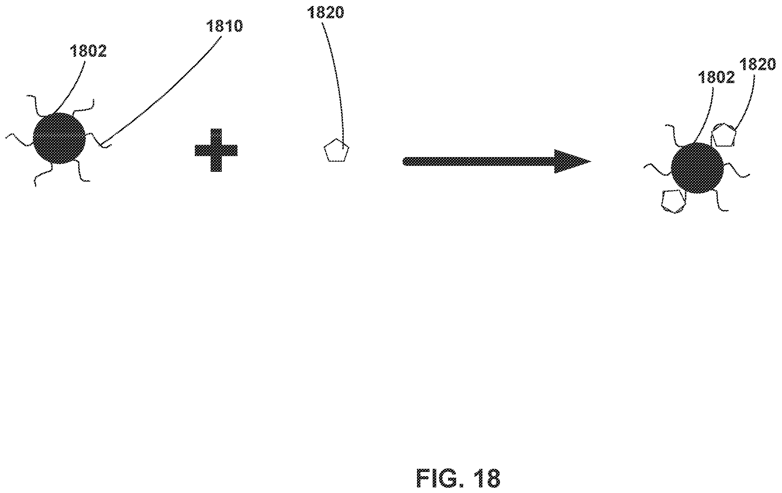

FIG. 18 is an illustration of an example surface functionalized MNP 1802, in accordance with one or more techniques of this disclosure. In the example shown, MNP 1802 may be surface functionalized via a coating of one or more single strand DNA and/or RNA 1810. In some examples, one or more DNA-binding proteins 1820 may interact and/or bind to the one or more single strand DNA and/or RNA 1810, for example, via addition of a biofluid containing DNA-binding proteins 1820 to sample vial 108 including MNPs 1802. In some examples, MNP 1802 may have an increased hydrodynamic size as a result of the interaction/binding of DNA-binding proteins 1820 with single strand DNA and/or RNA 1810. In some examples, DNA-binding proteins 1820 may be one or more analytes, for example, DNA-binding proteins 1820 may be one or more analytes of a coronavirus such as human coronavirus 229E, human coronavirus OC43, SARS-CoV (2003), HCoV NL63 (2004), HKU1 (2005), MERS-CoV (2102), SARS-CoV-2, and the like. In some examples, DNA-binding proteins 1820 may be any of a membrane, an envelope, a structure protein, a Hemagglutinin esterase protean, a nucleocapsid protein inside the envelop, and the like, of a coronavirus. In some examples, DNA-binding proteins 1820 may include transcription factors which modulate the process of transcription, various polymerases, nucleases which cleave DNA molecules, and histones involved in chromosome packaging and transcription in the cell nucleus.

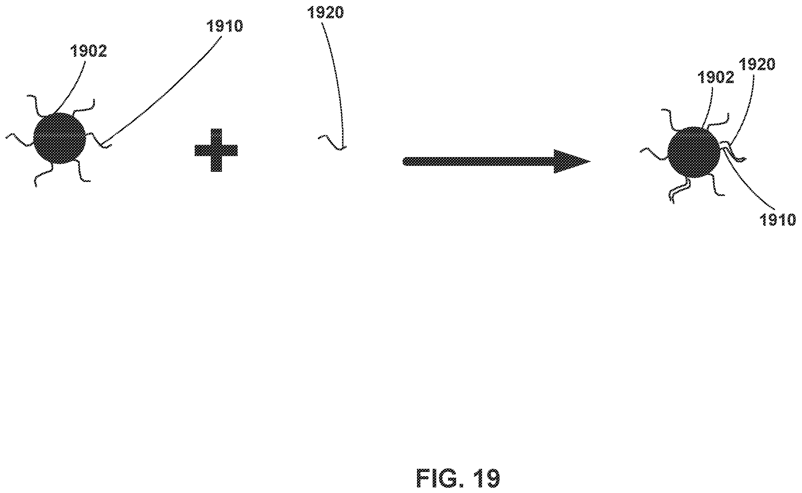

FIG. 19 is an illustration of an example surface functionalized MNP 1902, in accordance with one or more techniques of this disclosure. In the example shown, MNP 1902 may be surface functionalized via a coating of one or more reverse complementary single strand DNA and/or RNA 1910. In some examples, one or more single strand DNA and/or RNA 1920 may interact and/or bind to the one or more reverse complementary single strand DNA and/or RNA 1910, for example, via addition of a biofluid containing single strand DNA and/or RNA 1920 to sample vial 108 including MNPs 1902. In some examples, MNPs 1902 may have an increased hydrodynamic size as a result of the interaction/binding of single strand DNA and/or RNA 1920 with reverse complementary single strand DNA and/or RNA 1910. In some examples, single strand DNA and/or RNA 1920 may be one or more analytes, for example, single strand DNA and/or RNA 1920 may be one or more analytes of a coronavirus such as human coronavirus 229E, human coronavirus OC43, SARS-CoV (2003), HCoV NL63 (2004), HKU1 (2005), MERS-CoV (2102), SARS-CoV-2, and the like. In some examples, single strand DNA and/or RNA 1920 may be any of a single stranded DNA (ss-DNA) and/or RNA (ss-RNA), and the like, of a coronavirus.

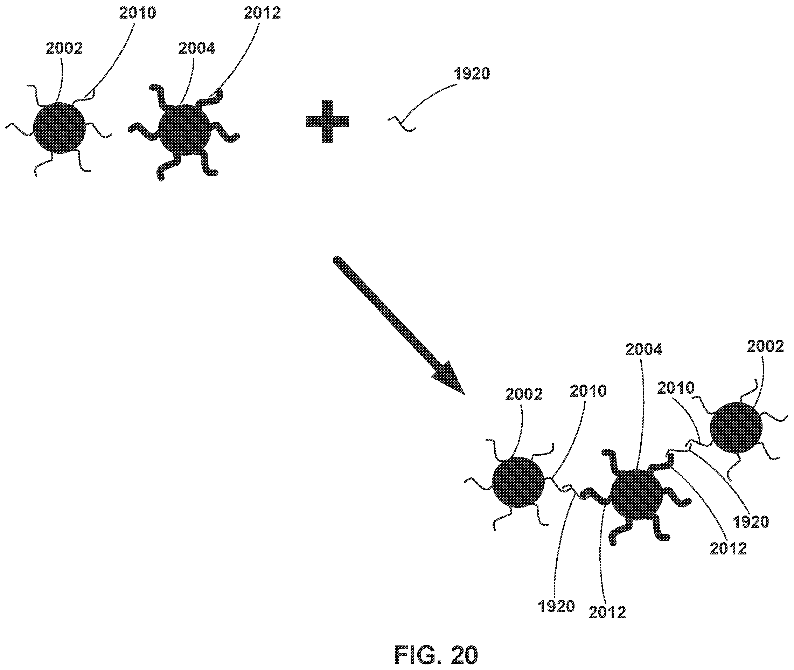

FIG. 20 is an illustration of example surface functionalized MNPs 2002 and 2004, in accordance with one or more techniques of this disclosure. In the example shown, MNPs 2002 and 2004 may be surface functionalized via a coating of one or more single strand DNA and/or RNA 2510 and 2012, respectively. In some examples, single strand DNA and/or RNA 2510 and 2012 may be different from each other, but still at least partially pair with different portions of one or more single strand DNA and/or RNA 1920. In some examples, one or more single strand DNA and/or RNA 1920 may interact, bind, and/or partially pair with the one or more single strand DNA and/or RNA 2510 and 2012, for example, via addition of a biofluid containing single strand DNA and/or RNA 1920 to sample vial 108 including MNPs 2002 and 2004. In the example shown, MNPs 2002 and 2004 may cluster as a result of the interaction/binding/at least partial pairing with single strand DNA and/or RNA 1920, and in some examples, MNPs 2002 and 2004 may have an increased hydrodynamic size as a result of the interaction/binding/at least partial pairing of single strand DNA and/or RNA 1920 with single strand DNA and/or RNA 2510 and 2012. In some examples, single strand DNA and/or RNA 1920 may be one or more analytes, for example, single strand DNA and/or RNA 1920 may be substantially similar to single strand DNA and/or RNA 1920 illustrated and described above with respect to FIG. 19.

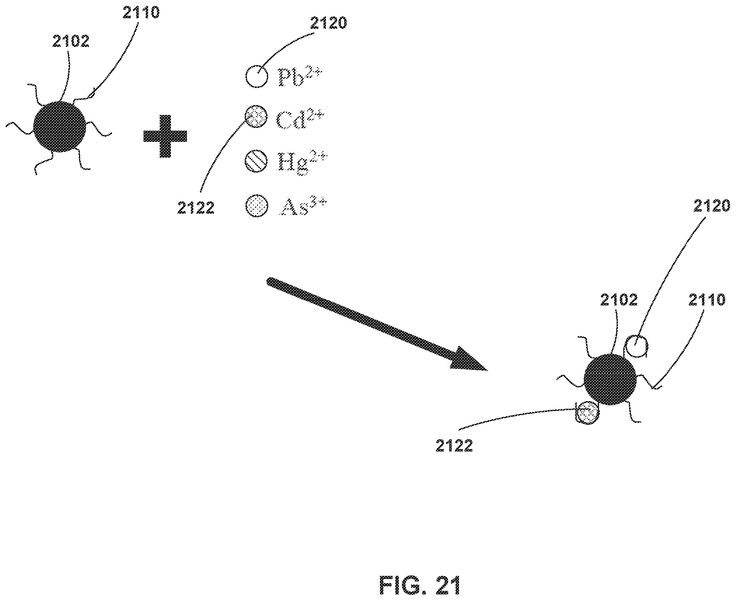

FIG. 21 is an illustration of an example surface functionalized MNP 2102, in accordance with one or more techniques of this disclosure. In the example shown, MNP 2102 may be surface functionalized via a coating of one or more differing types of structure switching strand DNA and/or RNA 2110. In some examples, one or more heavy metal ions 2120 and/or 2122 may interact and/or bind to the one or more differing types of structure switching strand DNA and/or RNA 2110, for example, via addition of a biofluid, drinking water, or any fluid containing heavy metal ions 2120 and/or 2122 to sample vial 108 including MNPs 2102. In some examples, MNPs 2102 may have an increased or decreased hydrodynamic size as a result of the interaction/binding of heavy metal ions 2120 and/or 2122 with the one or more differing types of structure switching strand DNA and/or RNA 2110.

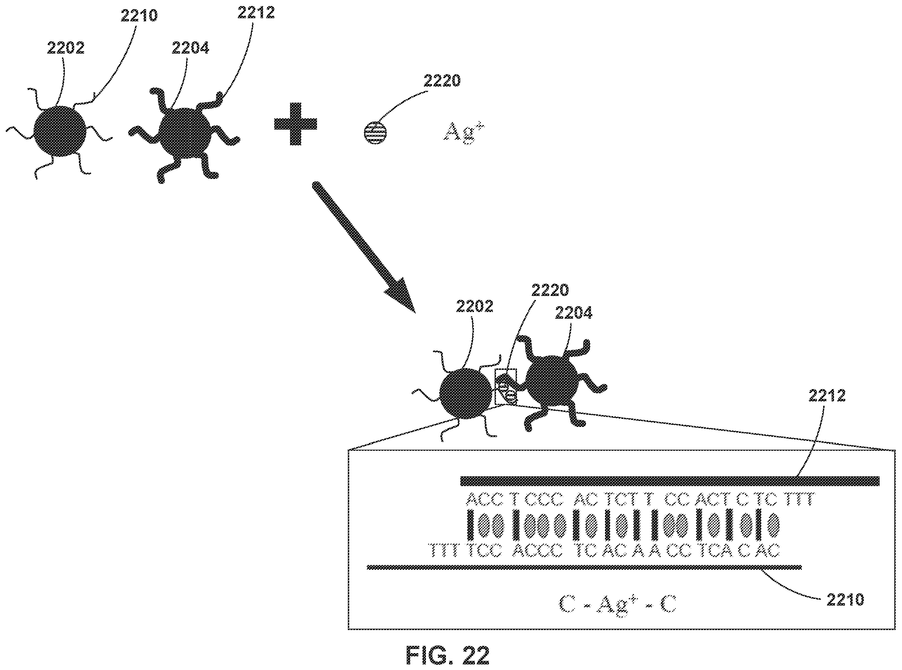

FIG. 22 is an illustration of example surface functionalized MNPs 2202 and 2204, in accordance with one or more techniques of this disclosure. In the example shown, MNPs 2202 and 2204 may be surface functionalized via a coating of one or more single strand DNA and/or RNA 2210 and 2212, respectively. In some examples, single strand DNA and/or RNA 2210 and 2212 may be different from each other may include partially mismatched single strand RNA and/or DNA. In some examples, one or more heavy metal ions 2220 may interact, bind, and/or at least partially match with the one or more single strand DNA and/or RNA 2010 and 2012, for example, via addition of a biofluid, drinking water, etc., containing one or more heavy metal ions 2220 to sample vial 108 including MNPs 2202 and 2204. In the example shown, MNPs 2202 and 2204 may cluster as a result of the interaction/binding/at least partial matching with heavy metal ions 2220, and in some examples, MNPs 20202 and 2204 may have an increased hydrodynamic size as a result of the interaction/binding/at least partial matching of heavy metal ions 2220 with single strand DNA and/or RNA 2210 and 2212.

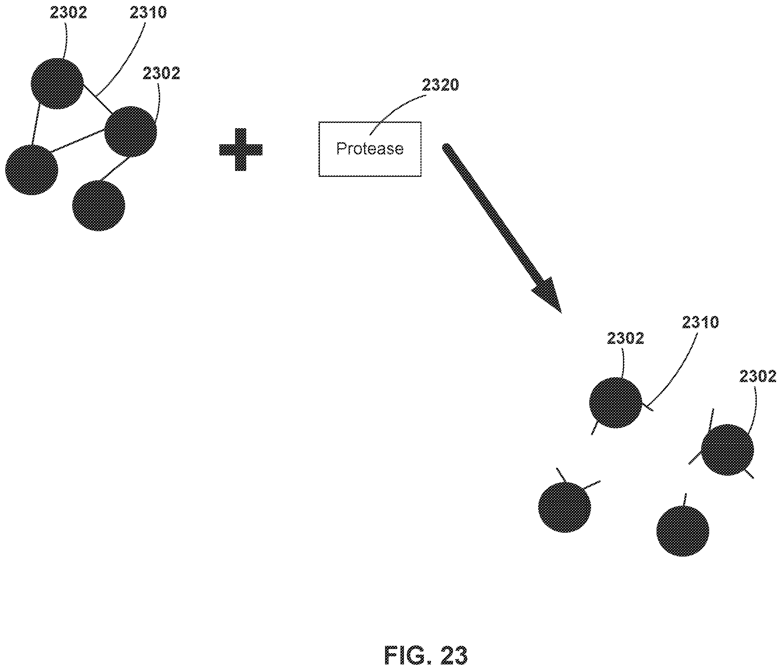

FIG. 23 is an illustration of an example surface functionalized MNPs 2302, in accordance with one or more techniques of this disclosure. In the example shown, a plurality of MNPs 2302 may be interlinked via one or more peptides 2310. In some examples, one or more target proteases 2320 may interact and or cleave peptides 2310, for example, via addition of a biofluid to sample vial 108 including MNPs 2302, thereby reducing the hydrodynamic size of the interlinked MNPs 2302. The presence of the target protease 2320 may result in a decreased hydrodynamic size of clusters of NMPs 2302 due to cleaving of peptides. For example, proteases 2320 may regulate multiple biological processes including cell differentiation, proteasomal degradation, inflammation, tissue remodeling, apoptosis, cell homeostasis, and coagulation. Consequently, the deregulation of proteolytic activity accounts for pathogenesis and progression of many diseases such as cardiovascular disease, inflammatory conditions, neurodegenerative disorders and cancer.

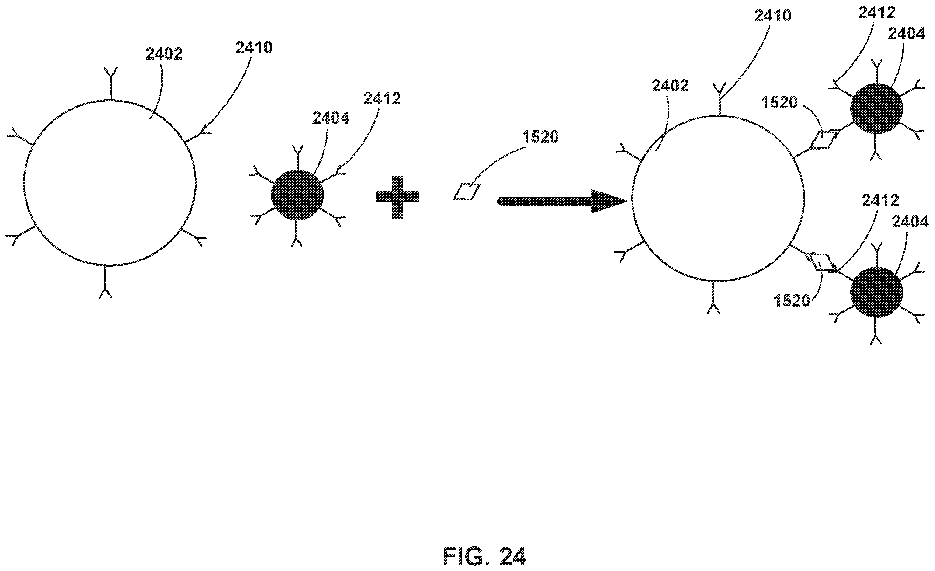

FIG. 24 is an illustration of example surface functionalized microbead 2402 and MNP 2404, in accordance with one or more techniques of this disclosure. In the example shown, nonmagnetic microbead 2402 may be surface functionalized via a coating of one or more antibodies 2410, and MNP 2404 may be surface functionalized via a coating of one or more antibodies 2412, where antibodies 2410 may be different from antibodies 2412. In some examples, microbead 2402 may be made of a nonmagnetic material, e.g., a polymer, polystyrene, or any suitable nonmagnetic material. In some examples, one or more antigens 1520 may bind to either of the one or more antibodies 2410 and 2412, for example, via addition of a biofluid containing antigens 1520 to sample vial 108 including MNPs 2402 and 2404. In the example shown, microbead 2402 and MNP 2404 may cluster as a result of antibodies 2410 and 2412 binding with antigens 1520, and in some examples, microbead 2402 and MNP 2404 may have an increased hydrodynamic size as a result of the binding of antigens 1520 to antibodies 2410 and 2412. In some examples, antigens 1520 may be one or more analytes, for example, antigens 1520 may be one or more analytes of a coronavirus such as human coronavirus 229E, human coronavirus OC43, SARS-CoV (2003), HCoV NL63 (2004), HKU1 (2005), MERS-CoV (2102), SARS-CoV-2, and the like. In some examples, antigens 1520 may be any of a membrane, an envelope, a structure protein, a Hemagglutinin esterase protean, a nucleocapsid protein inside the envelop, and the like, of a coronavirus. In some examples, microbead 2402 may replace one of the types of MNP 1602 and 1604 in the example shown in FIG. 16 above. In some examples, antigen 1520 may be substantially similar to antigen 1520 illustrated and described above with respect to FIG. 15.

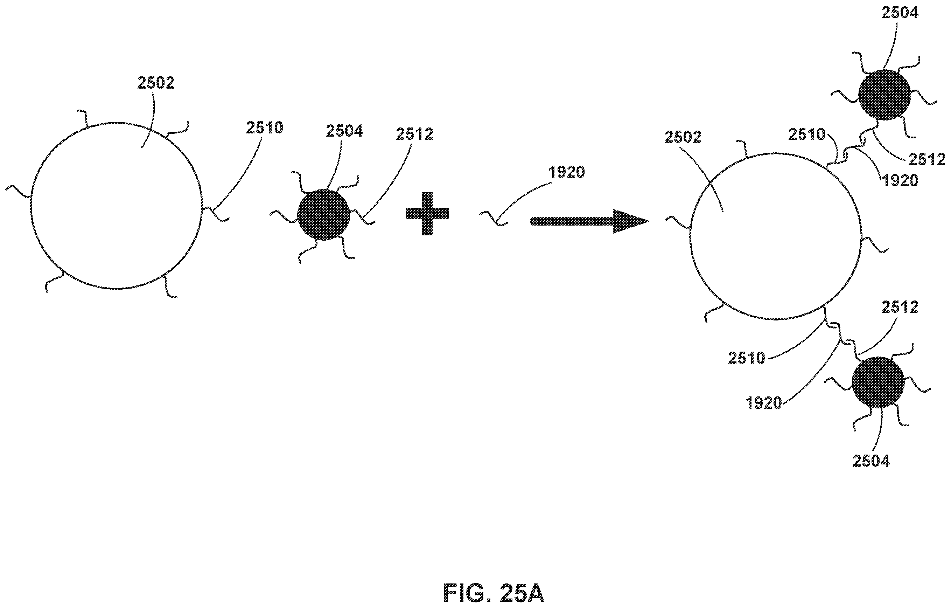

FIG. 25A is an illustration of example surface functionalized nonmagnetic microbead 2502 and MNP 2504, in accordance with one or more techniques of this disclosure. In the example shown, microbead 2502 and MNP 2504 may be surface functionalized via a coating of one or more single strand DNA and/or RNA 2510 and 2512, respectively. In some examples, single strand DNA and/or RNA 2510 and 2512 may be different from each other, but still at least partially pair with one or more single strand DNA and/or RNA 1920. In some examples, one or more single strand DNA and/or RNA 1920 may interact, bind, and/or partially pair with the one or more single strand DNA and/or RNA 2510 and 2512, for example, via addition of a biofluid containing single strand DNA and/or RNA 1920 to sample vial 108 including microbeads 2502 and MNPs 2504. In the example shown, microbeads 2502 and MNPs 2504 may cluster as a result of the interaction/binding/at least partial pairing with single strand DNA and/or RNA 1920, and in some examples, microbeads 2502 and MNPs 2504 may have an increased hydrodynamic size as a result of the interaction/binding/at least partial pairing of single strand DNA and/or RNA 1920 with single strand DNA and/or RNA 2510 and 2512. In some examples, single strand DNA and/or RNA 1920 may be one or more analytes, for example, single strand DNA and/or RNA 1920 may be substantially similar to single strand DNA and/or RNA 1920 illustrated and described above with respect to FIG. 19.In some examples, microbead 2502 may replace one of the types of MNP 2002 and 2004 in the example shown in FIG. 20 above.

Additionally, in some examples, microbeads 2402, 2502 may replace one of the types of MNP 2202, 2204 as described and illustrated above with respect to FIG. 22, and/or replace some of or be added to MNPs 2302 as described and illustrated above with respect to FIG. 23. Replacement of one of the types of MNP 1502, 1602, 2002, 2004, 2202, 2204, and 2302 by microbeads 2402 and/or 2502 in the examples described above with respect to FIGS. 16, 20, 22-25, respectively, may increase hydrodynamic size, and thereby may increase the sensitivity of MPS measurements.

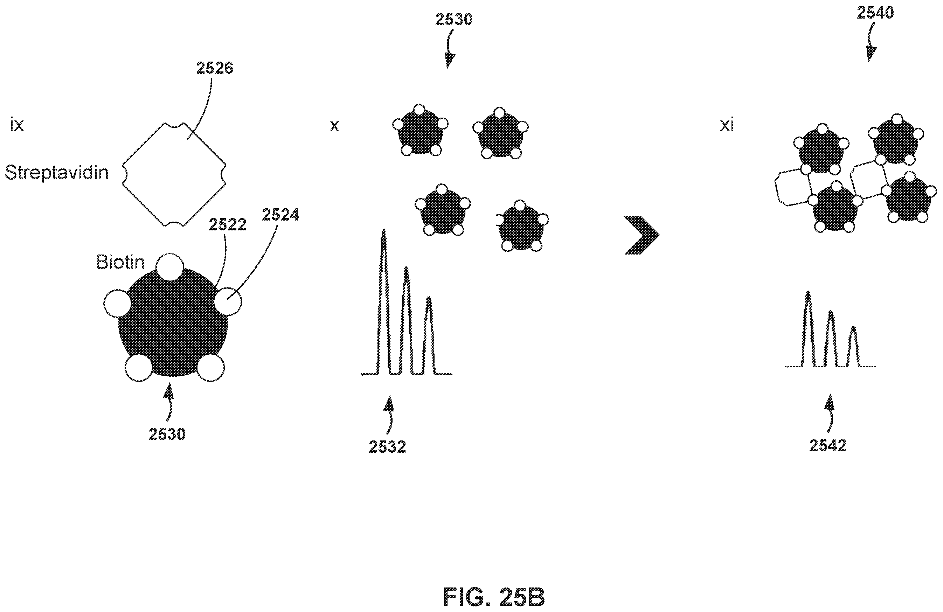

FIG. 25B is an illustration of example surface functionalized MNP 2522, in accordance with one or more techniques of this disclosure. In the example shown, MNP 2522 may be surface functionalized via a coating of biotin 2524. Biotin 2524 may be selected to bind to one or more streptavidin protein 2526.

Streptavidin is a homo-tetramer with an extraordinarily high affinity for biotin, e.g., 1 mol of streptavidin can bind with 4 mol of biotin. In the example shown, well-dispersed biotinylated MNPs 2530 show high dynamic magnetic responses to external oscillating fields as well as large harmonic amplitudes 2532. In the presence of streptavidin 2526, biotinylated MNPs 2530 may cross-link and form clusters 2540 on streptavidin homo-tetramers. The clustering of MNPs may weaken the dynamic magnetic responses, and as a result, the harmonic amplitudes drop and/or reduce relative to well-dispersed MNPs, e.g., illustrated as harmonic amplitudes 2542 reduced relative to harmonic amplitudes 2532 in the example shown. The difference in harmonic amplitude reduction may be used to quantitatively analyze the amount and concentration of streptavidin in a sample. An MPS-based bioassay, e.g., such as with MPS handheld device 102, may not require removing unbound target analytes (e.g., streptavidin 2526 in the example shown) and may be a wash-free, one-step test that is accessible by a layperson in nonclinical settings.

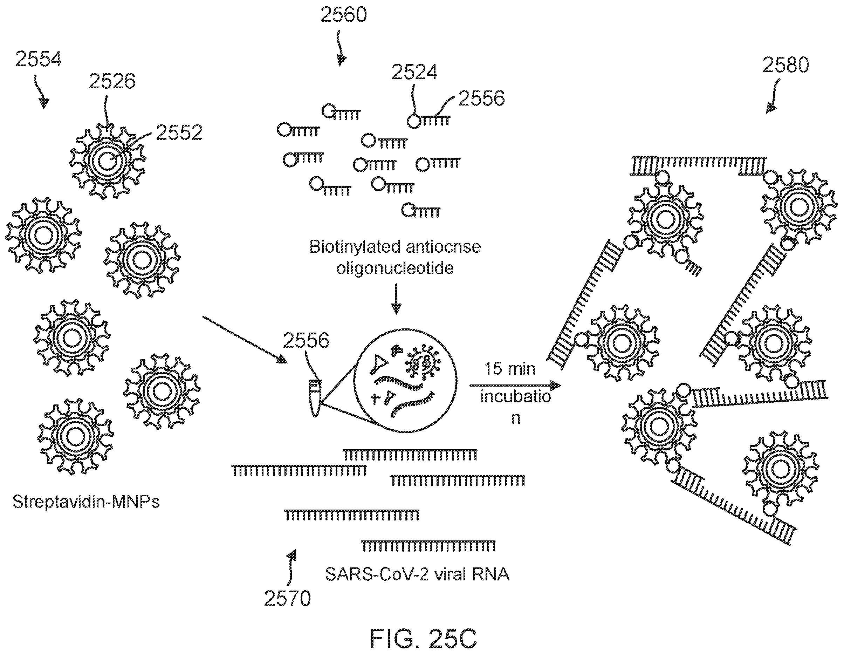

FIG. 25C is an illustration of example surface functionalized MNPs 2552, in accordance with one or more techniques of this disclosure. In the example shown, MNP 2522 may be surface functionalized via a coating of biotin 2524 and streptavidin proteins 2526, e.g., which may form streptavidin-saturated biotinylated MNPs 2554. For example, a significant portion of biotin 2524 of each MNP 2526 may bind with streptavidin proteins 2526 individually, e.g., such that each MNP 2554 does not form clusters such as clusters 2540 of FIG. 25B, but may remain dispersed because a threshold amount of the biotin, or substantially all of the biotin, of the surface functionalized MNPs 2554 may already be bound to streptavidin proteins 2526.

In some examples, one or more antisense oligonucleotides 2556 may be bound to biotin 2524 to form biotinylated antisense oligonucleotides 2560 and introduced to streptavidin-saturated biotinylated MNPs 2554, e.g., alone or in combination with a biological sample including analytes 2570 of a coronavirus such as human coronavirus 229E, human coronavirus OC43, SARS-CoV (2003), HCoV NL63 (2004), HKU1 (2005), MERS-CoV (2102), SARS-CoV-2, and the like. For example, analyte 2570 may be a SARS-CoV-2 viral RNA. In some examples, streptavidin-saturated biotinylated MNPs 2554, biotinylated antisense oligonucleotides 2560, and analytes 2570 may be combined and/or mixed biological sample vial 2556.

In some examples, the biotinylated antisense oligonucleotides 2560 may be configured to bind and/or pair with analytes 2570, and the streptavidin-saturated biotinylated MNPs 2554, biotinylated antisense oligonucleotides 2560, and analytes 2570 may form clusters 2580. The clustering of MNPs may weaken the dynamic magnetic responses, and as a result, the harmonic amplitudes drop and/or reduce relative to well-dispersed MNPs. The difference in harmonic amplitude reduction may be used to quantitatively analyze the amount and concentration of analyte 2570 in a sample. An MPS-based bioassay, e.g., such as with MPS handheld device 102, may not require removing unbound target analytes 2570 and may be a wash-free, one-step test that is accessible by a layperson in nonclinical settings.

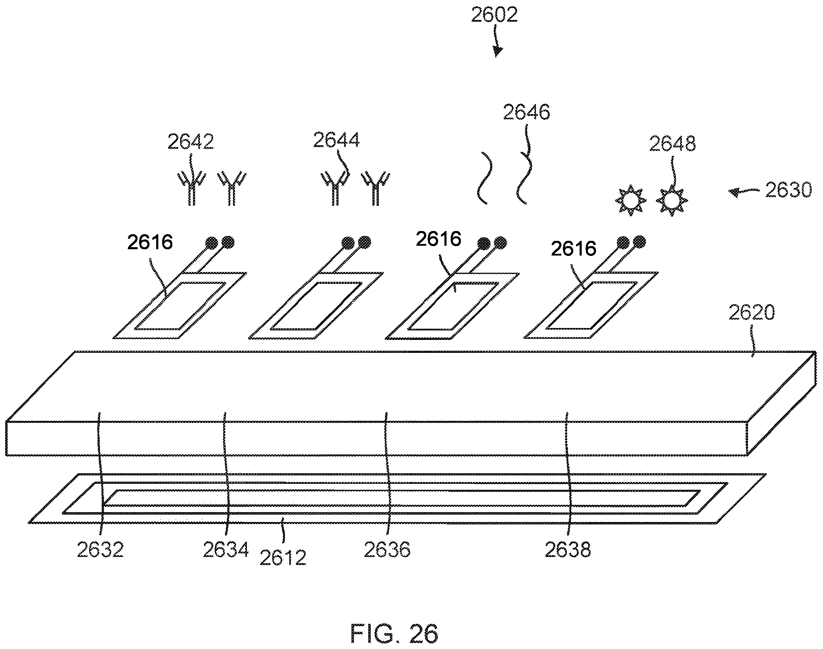

FIGS. 26-29 are diagrams illustrating examples of altered form MPS devices 2602 and 2702 and an example method 2900 of measuring a sample using MPS devices 2602 and 2702. In some examples, MPS devices 2602 and 2702 may use surface-bound antigens, antibodies, single strand DNA and/or RNA, and the like.

FIG. 26 is an illustration of an exploded-view of an example MPS device 2602, in accordance with one or more techniques of this disclosure. In the example shown, MPS device 2602 includes excitation coils 2612, sensing and/or pick-up coils 2616, testing strip 2620, and a chemical functionalization layer 2630. In some examples, MPS device 2602 may include a testing strip, and may contain one or more sensing regions, each region specific to a target analyte. In some examples, MPS device 2602 may be compatible with, or built into, a microfluidic channel and combined with system providing samples to test via microfluidic channels, e.g., a blood filtration system.

In the example shown, excitation coils 2612 are located on or adjacent to the bottom surface of testing strip 2620. Excitation coils 2612 may be substantially similar in function to excitation coils 1212 and/or 1214 illustrated and described above with respect to FIG. 12 and may include one or more windings similar to excitation coils 1212 and 1214. However, as shown in FIG. 26, excitation coils 2612 are substantially planar.

In the example shown in FIG. 26, chemical functionalization layer 2630 may include a plurality of sensing regions, e.g., sensing regions 2632-2638. Each sensing region may include a surface functionalization coating including a corresponding probe type, for example, a first antibody 2642, a second antibody 2644 different from first antibody 2642, single stranded DNA or RNA 2646, and antigen 2648. As such, each sensing region may detect a different analyte. Each of the plurality of probes may be configured for detecting, binding with, etc., a specific type of analyte. In some examples, chemical functionalization layer 2630 may be disposed on top of sensing coils 2616, each sensing coil 2616 corresponding to a sensing region 2632-2638.

In the example shown, sensing coils 2616 are located on the top surface of testing strip 2620, and may be substantially similar to pick-up coils 1216 illustrated and described above with respect to FIG. 12, aside from their substantially planar configuration. In the example shown, MPS device 2602 includes a sensing coil 2616 corresponding to each of the plurality of sensing regions, e.g., sensing regions 2632-2638, for example, to independently measure analytes being tested for corresponding to each of the plurality of regions.

In the example shown testing strip 2620 may provide structure for MPS device 2602 and a flow path for a biofluid to come in fluid communication with surface functionalization layer 2630. In some examples, testing strip 2620 may be and/or include a nitrocellulose membrane.

In the example shown, each probe type 2642-2648 of sensing region 2632-2638 may bind to a specific analyte type, and each bound analyte type may further bind to a surface functionalized MNP. For example, a fluid including a biologic sample including one or more analytes and one or more surface functionalized MNPs may be fluidically provided to testing strip 2620 and may come in fluid communication with surface functionalization layer 2630. The analytes corresponding to the specific probe types 2642-2648 may be bound and captured by the probes 2642-2648, and MNPs with surface functionalization corresponding to the captured analytes may be bound and captured to the captured analytes. In some examples, an alternating magnetic field may be generated by excitation coils 2612, and sensing coils 1610 may detect the magnetic response of the captured MNPs and generate a signal based on the magnetic response of the captured MNPs.

In some examples, MPS device 2602 may detect the magnetic moment of captured MNPs, e.g., sensing coil 2616 may generate a signal proportional to the magnetic moment of one or more captured MNPs within the alternating magnetic field. For example, MPS device 2602 may generate a signal including harmonics of the frequencies of the excitation coils, and the presence of the harmonics may be proportional to the magnetic moment of the plurality of captured MNPs. Because the motion of MNPs due to the alternating magnetic field is restricted via being bound to a surface of MPS device 2602, the magnetic response of MNPs of MPS device 2602 bound to the surface may not undergo Brownian motion and may not obey a Langevin function, e.g., in contrast to the operating principle of MPS handheld device 102 described above with respect to FIG. 13. In some examples, the MNPs of MPS device 2602 may be single-core or multi-core, and may be superparamagnetic.

In some examples, MPS device 2602 may communication with a computing device or devices, e.g., computing device 104, similar to MPS handheld device 102 as illustrated and described above with respect to FIG. 1, and may output a signal to computing device 104.

In some examples, MPS device 2602 may be a double-layered printed circuit board (PCB), or integrated into a double-layered PCB, or MPS device 2602 may be, or may be integrated into, a silicon substrate and/or a polymer substrate, e.g., via microfabrication. For example, coils 2612 and 1610 may be integrated into a double-layered PCB or micro-fabricated on a silicon substrate, and MPS device 2602 may be an adaptor card configured to be inserted into, and communicatively coupled to, a different MPS device, e.g., MPS device 3004 described and illustrated below, and/or a computing device.

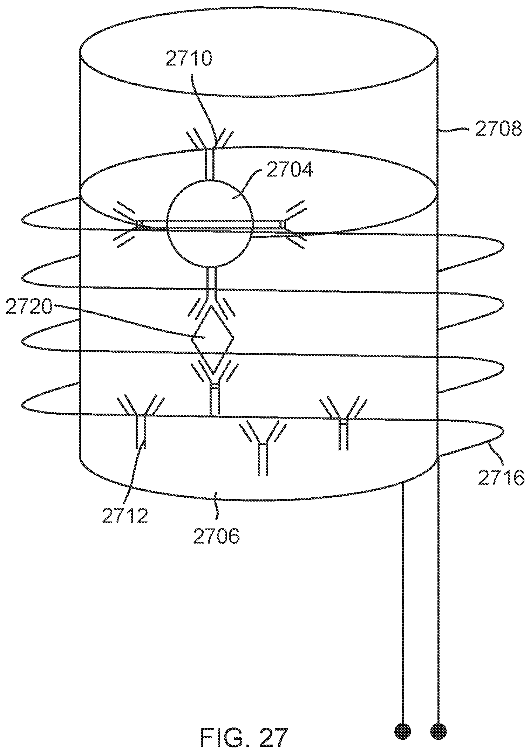

FIG. 27 is an illustration of an example MPS device 2702, in accordance with one or more techniques of this disclosure. In the example shown, MPS device 2702 includes coils 2716 and container 2708. In some examples, MPS device 2702 may be compatible with, or built into, a microfluidic channel and combined with a system providing samples to test via microfluidic channels, e.g., a blood filtration system.

In the example shown, coils 2716 are configured to coil around container 2708. Although only one set of coils is illustrated, coils 2716 may include primary, secondary, etc., excitation coils and one or more sets of sensing coils, e.g., pick-up coils. The excitation coils may be substantially similar to excitation coils 1212 and/or 1214 illustrated and described above with respect to FIG. 12, and may include one or more windings similar to excitation coils 1212 and 1214. The sensing coils may be substantially similar to pick-up coils 1216 illustrated and described above with respect to FIG. 12.

In the example shown, container 2708 includes a chemical functionalization layer 2706. Chemical functionalization layer 2706 may be bound to one or more inner surfaces of container 2708, and may include a surface functionalization coating including a plurality of probe types, for example, antibodies, antigens, single stranded DNA or RNA, and the like. In the example shown, chemical functionalization layer 2706 is coated with antibodies 2712. Each of the plurality of probes, e.g., antibodies 2712, may be configured for detecting, binding with, etc., a specific type of analyte.

In the example shown, MPS device 2702 includes a plurality of MNPs, e.g., MNP 2704. MNP 2704 may be surface functionalized via a coating of a probe type, e.g., antibodies, antigens, single stranded DNA or RNA, and the like. In the examples shown, MNP 2704 is surface functionalized via a coating of antibodies 2710. In some examples, antibodies 2712 may correspond to antibodies 2710, that is, antibodies 2710 and 2712 may be configured for detecting, binding with, etc., the same specific type of analyte.

In operation, a biological sample may be added to MPS device 2702 including an analyte, e.g., an antibody, antigen, single stranded DNA or RNA, and the like. In the example shown, antigen 2720 has been added to container 2708. In some examples, the antibodies 2710 and 2712 may bind with antigen 2720, thereby binding MNP 2704 to chemical functionalization layer 2706.

In some examples, MPS device 2702 may communication with a computing device or devices, e.g., computing device 104, similar to MPS handheld device 102 as illustrated and described above with respect to FIG. 1. In some examples, the operating principles of MPS device 2702 may be similar to that illustrated described above with respect to FIGS. 12-13.

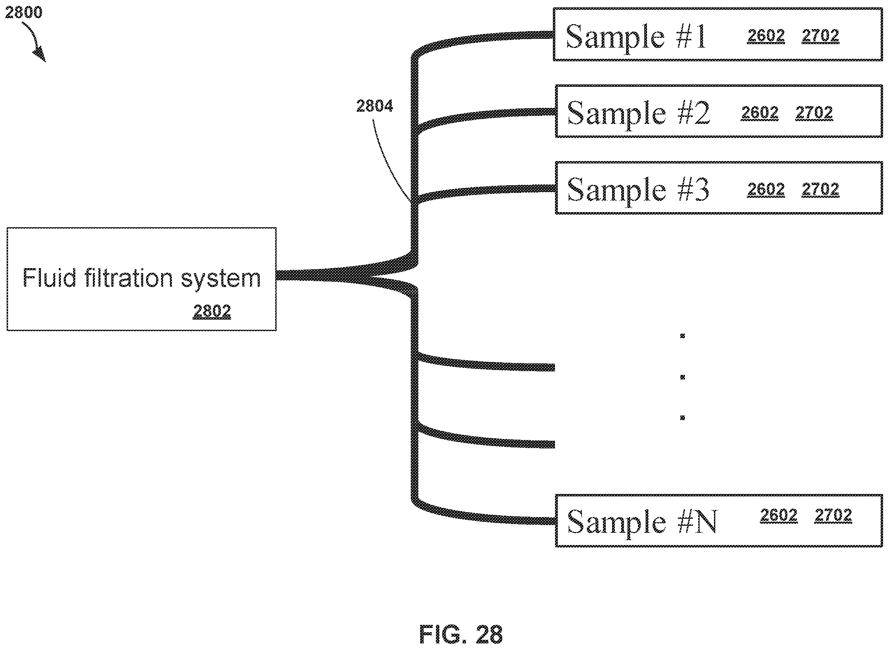

FIG. 28 is an illustration of an example microfluidic system 2800 that may be used with an MPS device, in accordance with one or more techniques of this disclosure. For example, microfluidic system 2800 may be used with MPS devices 2602 and/or 2702. In the example shown, microfluidic device 2800 includes fluid filtration system 2802 and a plurality of microfluidic channels 2804.

In some examples, fluid filtration system 2802 may be configured to filter a fluid, e.g., a patient's blood, and to transfer the filtered fluid and/or biological samples to microfluidic channels 2804. Microfluidic channels 2804 may be configured to transport filtered fluid and/or biological samples to a plurality of MPS devices, e.g., MPS devices 2602 and/or 2702.

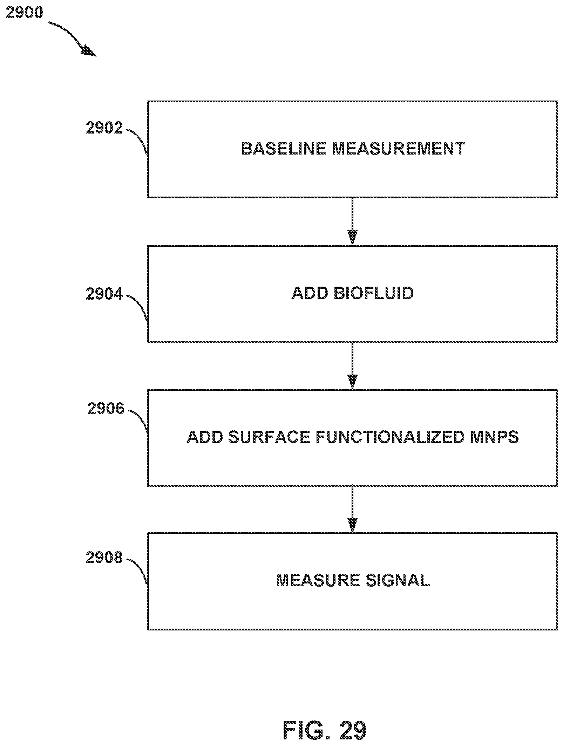

FIG. 29 is a flowchart of an example method 2900 of measuring a sample using MPS devices 2602 and/or 2702, in accordance with one or more techniques of this disclosure. The example method is described with reference to FIGS. 26 and 27 above. The example method may be performed, for example, by a computing device, such as computing device 104 and/or distributed computing device 106, executing the steps of the method.

Excitation coils 2612 and/or excitation coils of coils 2716 may generate magnetic fields, and sensing coils 2616 and/or sensing coils of coils 2716 may measure a baseline signal (2902). A biofluid sample may flow across testing strip 2620 and chemical functionalization layer 2630, or a biofluid sample may flow into container 2708 (2904). In some examples, target analytes specific to the probes coated in each of regions 2632-2638 may be captured in each region, and the target analytes may be anchored to the top surface of testing strip 2620 by the coated probes. In other examples, target analytes specific to the probes coated on chemical functionalization layer 2706 may be captured and anchored to chemical functionalization layer 2706. Surface functionalized MNPs may flow across testing strip 2620 and chemical functionalization layer 2630 (2906). The MNPs may include any of the surface functionalized MNPs illustrated and described above with respect to FIGS. 15-23, e.g., the MNPs may be functionalized via surface coatings of antibodies, antigens, single stranded DNA or RNA, and the like. The surface functionalized MNPs may be captured via the captured target analytes in the plurality of regions, e.g., regions 2632-2638, corresponding to the surface functionalization of the individual MNPs. For example, an antigen may capture an antibody surface functionalized MNP at regions 2632, a different antigen may capture an MNP surface functionalized with a different antibody corresponding to the different antigen at region 2634, a single stranded DNA or RNA surface functionalized MNP may be captured by a corresponding single stranded DNA or RNA at region 2636, and secondary antibody surface functionalized MNP may be captured by an antibody from the biofluid by an antigen at region 2638. In other examples, surface functionalized MNPs, e.g., MNPs 2704, may be introduced into container 2708 and may come into contact with chemical functionalization layer 2706. In some examples, differing antigens, antibodies, single stranded DNA or RNA and the like may be captured via a plurality of MPS devices 2702, each MPS device 2702 having a chemical functionalization layer of a specific probe type and in fluidic communication with a microfluidic channel of a sample providing system, e.g., microfluidic channel 2804 of microfluidic system 2800 Excitation coils 2612 and/or excitation coils of coils 2716 may generate magnetic fields, periodically magnetizing the captured MNPs (2708). The magnetic responses of the MNPs to the generated magnetic fields may be detected by sensing coils 2616 and/or sensing coils of coils 2716. Sensing coils 2616, and/or sensing coils of coils 2716, may generate a signal based on the detected magnetic responses of the MNPs, e.g., as illustrated and described above with respect to FIGS. 12-13.

In some examples, method 2900 may include additional steps, or may be performed in a different order, e.g., for measuring target proteases. In this scenario, MNPs surface functionalized with peptides may be added first. The MNPs may be captured in one or more regions correspondingly functionalized on testing strip 2620, or on chemical functionalization layer 2706. Baseline measurement may occur next, followed by an add biofluid sample step, e.g., a biofluid containing target proteases that may cleave the peptides. This may then be followed by a capture measurement step, with a positive result being indicated by the opposite magnetic response, e.g., measuring the lack of MNPs rather than their presence.

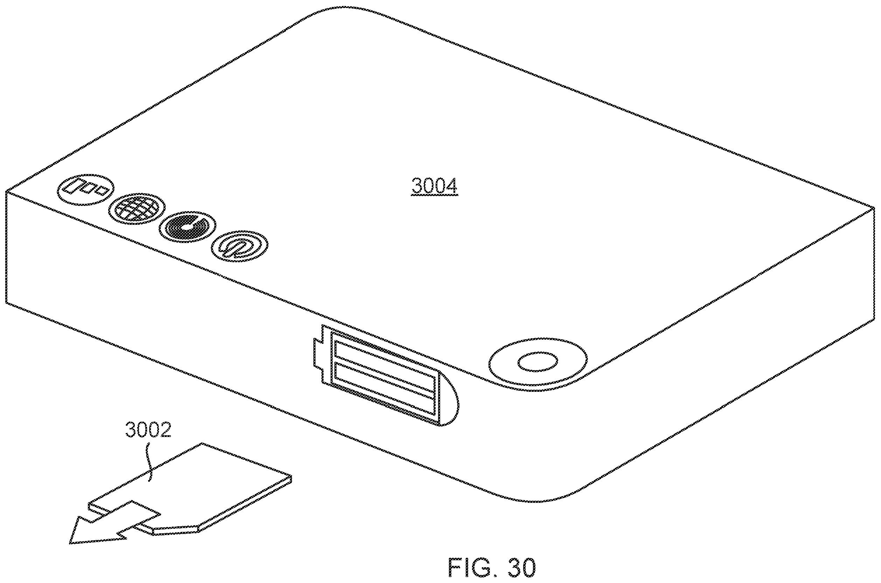

FIG. 30 is an illustration of example MPS devices 3002 and 3004, in accordance with one or more techniques of this disclosure. In the example shown, MPS device 3002 may be configured to be an adaptor card configured to be inserted into, and communicatively coupled to, a different MPS device, e.g., MPS device 3004, and/or a computing device.

The techniques described in this disclosure may be implemented, at least in part, in hardware, software, firmware or any combination thereof. For example, various aspects of the described techniques may be implemented within one or more processors, including one or more microprocessors, DSPs, application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit comprising hardware may also perform one or more of the techniques of this disclosure.

Such hardware, software, and firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components or integrated within common or separate hardware or software components.

The techniques described in this disclosure may also be embodied or encoded in a computer-readable medium, such as a computer-readable storage medium, containing instructions. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

## Claims

1. A bioassay system comprising:

   a conductive excitation coil (2612) configured to generate an alternating magnetic field (1310) including at least one of at least one sinusoidal wave, a square wave, a triangular wave, a sawtooth wave, or a combination of sinusoids thereof and apply the alternating magnetic field to a first sample region (2632) and a second sample region (2634);
   wherein the first sample region comprises:

   a first surface within the first sample region, the first surface coated with at least one probe (2642) configured to capture a first analyte type; and
   a first conductive sensing coil (2616) configured to determine a magnetic response of a surface functionalized magnetic nanoparticle, i.e. MNP, configured to be captured by the first analyte type;

   wherein the second sample region comprises:

   a second surface within the second sample region, the second surface coated with at least one probe (2644) configured to capture a second analyte type;
   a second conductive sensing coil (2616) configured to determine a magnetic response of a surface functionalized MNP configured to be captured by the second analyte type.

2. The bioassay system of claim 1, wherein the first and second sample regions are disposed on a microfluidic sample strip configured to allow a flow of a fluid containing at least one of at least one analyte and at least one surface functionalized MNP over the first and second surfaces, wherein the microfluidic sample strip is configured to be in fluid communication with a microfluidic device.

3. The bioassay system of claim 2, wherein the microfluidic device is configured to filter a fluid and deliver a filtered fluid including at least one analyte to a plurality of microfluidic channels, wherein each of the microfluidic channels are configured to be in fluid communication with a corresponding microfluidic sample strip, wherein the microfluidic device comprises one or more microfluidic mixing channels configured to mix a fluid including at least one surface functionalized MNP with the fluid including the at least one analyte, the one or more microfluidic mixing channels configured to be in fluidic communication with one or more of the plurality of microfluidic channels.

4. The bioassay system of any of claims 1 to 3, further comprising a processor configured to determine at least one harmonic amplitude and at least one ratio of harmonic amplitudes of the determined magnetic responses of the first and second regions.

5. The bioassay system of claim 4, wherein the processor is configured to determine the presence of the first analyte type and the second analyte type based on the at least one ratio of harmonic amplitudes, wherein the at least one ratio of harmonic amplitudes is independent of an MNP concentration.

6. The bioassay system of claim 5, further comprising a substrate defining the first and second opposing surfaces, wherein the conductive excitation coil is disposed on the first surface, wherein the first conductive sensing coil and the second conductive sensing coil are disposed on the second surface, wherein the substrate is one of silicon, a polymer, and a printed circuit board

**Patentansprüche**

1. Ein Bioassaysystem, das Folgendes beinhaltet:

eine leitfähige Erregerspule (2612), die konfiguriert ist, um ein magnetisches Wechselfeld (1310) zu erzeugen, das mindestens eines von mindestens einer Sinuswelle, einer Rechteckwelle, einer Dreieckswelle, einer Sägezahnwelle oder einer Kombination von Sinusoiden davon umfasst, und das magnetische Wechselfeld an einen ersten Probenbereich (2632) und einen zweiten Probenbereich (2634) anzulegen; wobei der erste Probenbereich Folgendes beinhaltet:

eine erste Oberfläche innerhalb des ersten Probenbereichs, wobei die erste Oberfläche mit mindestens einer Sonde (2642) beschichtet ist, die konfiguriert ist, um einen ersten Analyttyp einzufangen; und eine erste leitfähige Messspule (2616), die konfiguriert ist, um eine magnetische Reaktion eines oberflächenfunktionalisierten magnetischen Nanopartikels, d. h. MNP, zu bestimmen, das konfiguriert ist, um von dem ersten Analyttyp eingefangen zu werden;

wobei der zweite Probenbereich Folgendes beinhaltet:

eine zweite Oberfläche innerhalb des zweiten Probenbereichs, wobei die zweite Oberfläche mit mindestens einer Sonde (2644) beschichtet ist, die konfiguriert ist, um einen zweiten Analyttyp einzufangen; eine zweite leitfähige Messspule (2616), die konfiguriert ist, um eine magnetische Reaktion eines oberflächenfunktionalisierten MNP zu bestimmen, das konfiguriert ist, um von dem zweiten Analyttyp eingefangen zu werden.

2. Bioassaysystem gemäß Anspruch 1, wobei der erste und der zweite Probenbereich auf einem mikrofluidischen Probenstreifen angeordnet sind, der konfiguriert ist, um einen Fluss eines Fluids, das mindestens eines von mindestens einem Analyt und mindestens einem oberflächenfunktionalisierten MNP enthält, über die erste und die zweite Oberfläche zu ermöglichen, wobei der mikrofluidische Probenstreifen konfiguriert ist, um in Fluidkommunikation mit einer mikrofluidischen Vorrichtung zu sein.

3. Bioassaysystem gemäß Anspruch 2, wobei die mikrofluidische Vorrichtung konfiguriert ist, um ein Fluid zu filtern und ein gefiltertes Fluid, das mindestens einen Analyten umfasst, an eine Vielzahl von mikrofluidischen Kanälen abzugeben, wobei jeder der mikrofluidischen Kanäle konfiguriert ist, um in Fluidkommunikation mit einem entsprechenden mikrofluidischen Probenstreifen zu sein, wobei die mikrofluidische Vorrichtung einen oder mehrere mikrofluidische Mischkanäle beinhaltet, die konfiguriert sind, um ein Fluid, das mindestens ein oberflächenfunktionalisiertes MNP umfasst, mit dem Fluid, das den mindestens einen Analyten umfasst, zu mischen, wobei der eine oder die mehreren mikrofluidischen Mischkanäle konfiguriert sind, um in Fluidkommunikation mit einem oder mehreren der Vielzahl von mikrofluidischen Kanälen zu sein.

4. Bioassaysystem gemäß einem der Ansprüche 1 bis 3, das ferner einen Prozessor beinhaltet, der konfiguriert ist, um mindestens eine harmonische Amplitude und mindestens ein Verhältnis von harmonischen Amplituden der bestimmten magnetischen Reaktionen des ersten und des zweiten Bereichs zu bestimmen.

5. Bioassaysystem gemäß Anspruch 4, wobei der Prozessor konfiguriert ist, um das Vorhandensein des ersten Analyttyps und des zweiten Analyttyps basierend auf dem mindestens einen Verhältnis von harmonischen Amplituden zu bestimmen, wobei das mindestens eine Verhältnis von harmonischen Amplituden unabhängig von einer MNP-Konzentration ist.

6. Bioassaysystem gemäß Anspruch 5, das ferner ein Substrat beinhaltet, das die erste und die zweite gegenüberliegende Oberfläche definiert, wobei die leitfähige Erregerspule auf der ersten Oberfläche angeordnet ist, wobei die erste leitfähige Messspule und die zweite leitfähige Messspule auf der zweiten Oberfläche angeordnet sind, wobei das Substrat eines von Silizium, einem Polymer und einer Leiterplatte ist.

**Revendications**

1. Un système d'essai biologique comprenant :

une bobine d'excitation conductrice (2612) configurée pour générer un champ magnétique alternatif (1310) incluant au moins l'une d'entre au moins une onde sinusoïdale, une onde carrée, une onde triangulaire, une onde en dents de scie, ou une combinaison de sinusoïdes de celles-ci et appliquer le champ magnétique alternatif à une première région d'échantillon (2632) et une deuxième région d'échantillon (2634) ;
dans lequel la première région d'échantillon comprend :

une première surface au sein de la première région d'échantillon, la première surface étant revêtue d'au moins une sonde (2642) configurée pour capturer un premier type d'analyte ; et
une première bobine de détection conductrice (2616) configurée pour déterminer une réponse magnétique d'une nanoparticule magnétique, c'est-à-dire MNP, fonctionnalisée en surface configurée pour être capturée par le premier type d'analyte ;

dans lequel la deuxième région d'échantillon comprend :

une deuxième surface au sein de la deuxième région d'échantillon, la deuxième surface étant revêtue d'au moins une sonde (2644) configurée pour capturer un deuxième type d'analyte ;
une deuxième bobine de détection conductrice (2616) configurée pour déterminer une réponse magnétique d'une MNP fonctionnalisée en surface configurée pour être capturée par le deuxième type d'analyte.

2. Le système d'essai biologique de la revendication 1, dans lequel les première et deuxième régions d'échantillon sont disposées sur une bande d'échantillon microfluidique configurée pour permettre un écoulement d'un fluide contenant au moins un élément d'entre au moins un analyte et au moins une MNP fonctionnalisée en surface sur les première et deuxième surfaces, la bande d'échantillon microfluidique étant configurée pour être en communication fluidique avec un dispositif microfluidique.

3. Le système d'essai biologique de la revendication 2, dans lequel le dispositif microfluidique est configuré pour filtrer un fluide et apporter un fluide filtré incluant au moins un analyte à une pluralité de canaux microfluidiques, chacun des canaux microfluidiques étant configuré pour être en communication fluidique avec une bande d'échantillon microfluidique correspondante, le dispositif microfluidique comprenant un ou plusieurs canaux de mélange microfluidiques configurés pour mélanger un fluide incluant au moins une MNP fonctionnalisée en surface avec le fluide incluant l'au moins un analyte, les un ou plusieurs canaux de mélange microfluidiques étant configurés pour être en communication fluidique avec un ou plusieurs canaux de la pluralité de canaux microfluidiques.

4. Le système d'essai biologique de n'importe lesquelles des revendications 1 à 3, comprenant en outre un processeur configuré pour déterminer au moins une amplitude d'harmonique et au moins un rapport d'amplitudes d'harmoniques des réponses magnétiques déterminées des première et deuxième régions.

5. Le système d'essai biologique de la revendication 4, dans lequel le processeur est configuré pour déterminer la présence du premier type d'analyte et du deuxième type d'analyte sur la base de l'au moins un rapport d'amplitudes d'harmoniques, l'au moins un rapport d'amplitudes d'harmoniques étant indépendant d'une concentration de MNP.

6. Le système d'essai biologique de la revendication 5, comprenant en outre un substrat définissant les première et deuxième surfaces opposées, la bobine d'excitation conductrice étant disposée sur la première surface, la première bobine de détection conductrice et la deuxième bobine de détection conductrice étant disposées sur la deuxième surface, le substrat étant un élément d'entre du silicium, un polymère, et une carte de circuit imprimé.

FIG. 1

FIG. 2

FIG. 3

400

402 — PLACE MNP SAMPLE VIAL IN MPS DEVICE
AND MEASURE BASELINE SIGNAL

404 — ADD FLUID SAMPLE TO MNP SAMPLE VIAL

406 — WAIT FOR A PREDETERMINED TIME PERIOD
TO ALLOW CHEMICAL BONDING

408 — MEASURE SIGNAL

FIG. 4

**502**

MagiCoil
Sign-in

Email

Password

SIGN IN

—— OR ——

Don't have an
account? Create one

**504**

× Analyze sample

Select the disease
you are testing:

Virus ▾

Select the antigen you
are testing:

$H_3N_2$Influenza ▾

NEXT >

**506**

× Analyze sample

Bluetooth

Select MagicCoil below
to connect:

5AAA00000A7clb07T$ehX4RjArYf
-

PAIRED DEVICES
WILL-LAPTOP
DEH-X8500BH
BC02

**508**

× Analyze sample

Place the sample tube in
MagiCoil for a baseline
reading. Do not afdd bio-
fluid. Tap next when
ready.

NEXT >

**510**

× Analyze sample

Add the bio-fluid to the
sample tube and ensure
to replace it in MagiCoil.
Tap next when ready.

NEXT >

**512**

× Analyze sample

Record 1
Virus
$H_3N_2$ Influenza

f+3    f+5    f+7
Negative

FIG. 5

602

Plasma Collection Prior to Mixing

MNP Cartridge

Mixing Channels

Pump

Filtration Mixing

Red Path:
Green Path:

Blue Path

A

**FIG. 6**

Filter Clamp

Hydrophilic Micro Array

Micro Channel Leading to Pump

608

604

Blood Applied to Filter from Top with Pipette

Secondary Hole Added to Increase Plasma Yield

Diameter 0.8 inches

Filter

Dashed Red Path:

Shows Intended Path for Blood Plasma to Travel

FIG. 7

604

610

NOA 81 microchannels

FIG. 8

EP 4 136 439 B1

FIG. 9

Inlet 2

Inlet 1

0.015 X 0.015
(inches)

A

B

C

D

1004

Outlet

0   0.00175   0.0035   0.00525   0.007 (m)

FIG. 10

EP 4 136 439 B1

33

FIG. 11A

FIG. 11B

FIG. 12A

FIG. 12B

FIG. 12C

EP 4 136 439 B1

Nonlinear response of superparamagnetic nanoparticles (MNPs).

Magnetization induced from MNPs.

Odd harmonics found in collected signal:

$3^{rd}$ harmonic: $f_H \pm 2f_L$

$5^{th}$ harmonic: $f_H \pm 4f_L$

$7^{th}$ harmonic: $f_H \pm 6f_L$

...

Two driving fields, one with high frequency and the other with low frequency.

1340

1330

FIG. 13C

FIG. 13D

1350

FIG. 13E

FIG. 13B

FIG. 13A

1320

1310

FIG. 14A

EP 4 136 439 B1

FIG. 14B

FIG. 14C

FIG. 14D

FIG. 15

EP 4 136 439 B1

FIG. 16

FIG. 17

EP 4 136 439 B1

FIG. 18

EP 4 136 439 B1

FIG. 19

EP 4 136 439 B1

FIG. 20

FIG. 21

FIG. 22

EP 4 136 439 B1

FIG. 23

FIG. 24

FIG. 25A

FIG. 25B

FIG. 25C

FIG. 26

FIG. 27

FIG. 28

2900

2902 — BASELINE MEASUREMENT

2904 — ADD BIOFLUID

2906 — ADD SURFACE FUNCTIONALIZED MNPS

2908 — MEASURE SIGNAL

FIG. 29

FIG. 30

3004

3002

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63011702
- EP 3470832 A1
- EP 3581916 A1
- WO 2018144599 A1
- NL 632004